# EUROPEAN PATENT APPLICATION

(11) **EP 4 386 082 A1**
(43) Date of publication of application: **19.06.2024**
(21) Application number: 22855868.0
(22) Date of filing: 08.08.2022
(51) Int. Cl.: C12N 5/074, C12N 5/0735

(54) **METHOD FOR PRODUCING PLURIPOTENT STEM CELLS**

(30) Priority: 10.08.2021 JP 2021130903
(71) Applicant: Kaneka Corporation, Osaka-shi, Osaka 530-8288 (JP); RIKEN, Wako-shi, Saitama 351-0198 (JP)
(72) Inventor: KAMBAYASHI, Sho, Kobe-shi, Hyogo 650-0047 (JP); KAWAI, Yoshikazu, Kobe-shi, Hyogo 650-0047 (JP); HAYASHI, Yohei, Wako-shi, Saitama 351-0198 (JP); TAKASAKI, Mami, Wako-shi, Saitama 351-0198 (JP)
(74) Representative: Hoffmann Eitle
(86) International application number: PCT/JP2022/030290
(87) International publication number: WO 2023/017806

(57) **Abstract**

The death of some pluripotent stem cells in suspension culture of pluripotent stem cells is avoided. When performing the suspension culture of the pluripotent stem cells, a liquid medium that does not substantially contain a PKCβ inhibitor is used for a single-cell state and a liquid medium containing a PKCβ inhibitor and a WNT inhibitor is used after a cell mass is formed.

## Description

### Technical Field

The present invention relates to a method for producing pluripotent stem cells by suspension culture.

### Background Art

Pluripotent stem cells such as ES cells and iPS cells have the ability to grow indefinitely and the ability to differentiate into various somatic cells. The practical application of a treatment method that transplants somatic cells induced to differentiate from pluripotent stem cells has the potential to fundamentally transform treatment methods for intractable diseases and lifestyle-related diseases. For example, technologies have already been developed for inducing the differentiation of pluripotent stem cells into various somatic cells such as nerve cells, myocardial cells, blood cells, and retinal cells at the *in vitro* level.

Meanwhile, regenerative medicine using pluripotent stem cells still has issues to be addressed for practical use, and one of the issues is the productivity of pluripotent stem cells per se. For example, it is said that about 2 × 10¹¹ cells are required for liver regeneration. Methods for culturing pluripotent stem cells are roughly classified into adherent culture, in which cells are adhered to a flat culture substrate (plate) and cultured, and suspension culture, in which cells are cultured by suspending them in a liquid medium. To culture the above-described number of cells by the adherent culture, a culture substrate (plate) of 10⁶ cm² or more is required, which corresponds to about 20,000 ordinary 10-cm dishes. In this way, as the cell count obtained by the adherent culture on the culture substrate (plate) surface depends on the culture area, a vast area is required for scale-up, and it is challenging to supply cells in the amount necessary for regenerative medicine. Meanwhile, in the suspension culture, since cells are cultured while floating in a liquid medium, the cell count obtained depends on the volume of the medium. Therefore, it is thought that the scale-up of the suspension culture is relatively realistic, and it is suitable for the mass production of cells. For example, Non Patent Literature 1 discloses a suspension culture method of pluripotent stem cells while stirring a liquid medium using a spinner flask as a cell culture vessel for suspension culture.

In addition, another issue facing the practical application of regenerative medicine is the productivity of the target somatic cells. To efficiently produce target somatic cells, efforts have been made to improve the efficiency of differentiation induction by culturing high-quality pluripotent stem cells, and various methods have been reported. For example, Non Patent Literature 2 discloses a method for suppressing spontaneous differentiation of pluripotent stem cells by adding a protein kinase C (PKC) inhibitor to the medium in the adherent culture of pluripotent stem cells. In addition, Non Patent Literature 3 discloses a method for suppressing spontaneous differentiation of pluripotent stem cells by adding a tankyrase (TNKS) inhibitor to the medium in the adherent culture of pluripotent stem cells.

As described above, various methods have been developed to produce target somatic cells from pluripotent stem cells in the adherent culture.

### Citation List

### Non Patent Literature

Non Patent Literature 1: Olmer R. et al., Tissue Engineering: Part C, Volume 18 (10): 772-784 (2012)
Non Patent Literature 2: M. Kinehara et al., PLoS One. 2013; 8(1): e54122
Non Patent Literature 3: T. Sumi et al., PLoS One. 2013; 8(5): e63378

### Summary of Invention

### Technical Problem

As mentioned above, the suspension culture is suitable for mass production of pluripotent stem cells; however, it has been found that the suspension culture has the problem that unintended differentiation is more likely to occur than in the adherent culture. Therefore, the present inventors made attempts to develop a technology for suppressing the spontaneous differentiation into all three germ layers and maintaining the undifferentiated state of pluripotent stem cells by allowing a PKCβ inhibitor and a TNKS inhibitor, which is a type of WNT signal inhibitor, to constantly act in the suspension culture of the pluripotent stem cells. However, when a PKCβ inhibitor and a WNT signal inhibitor were added, a phenomenon in which some pluripotent stem cells died in the suspension culture was observed, which caused another problem, such as poor cell culture efficiency.

### Solution to Problem

As a result of conducting diligent studies to solve the above-described problems, the present inventors have found that when pluripotent stem cells are cultured by the suspension culture in a single-cell state, it is possible to avoid a decrease in cell culture efficiency without causing spontaneous differentiation with a medium containing a PKCβ inhibitor at the lowest possible concentration (i.e., a medium substantially free of the PKCβ inhibitor) at the stage before cell mass formation. This has led to the completion of the present invention.

The present invention encompasses the following.
(1) A method for producing pluripotent stem cells, comprising:
   a step of performing suspension culture of pluripotent stem cells in a single-cell state in a first liquid medium that does not substantially contain a PKCβ inhibitor; and a step of performing suspension culture of the pluripotent stem cells in a second liquid medium containing the PKCβ inhibitor and a WNT inhibitor after the pluripotent stem cells form a cell mass.
(2) The production method according to (1), which is characterized in that the step of performing the suspension culture in the second liquid medium is carried out by adding the PKCβ inhibitor to the first liquid medium after the pluripotent stem cells form the cell mass in the step of performing the suspension culture in the first liquid medium.
(3) The production method according to (1), which is characterized in that the first liquid medium is exchanged to the second liquid medium after the pluripotent stem cells form the cell mass.
(4) The production method according to any one of (1) to (3), which is characterized by further comprising a step of performing adherent culture of pluripotent stem cells and a step of detaching the pluripotent stem cells after the adherent culture and processing the pluripotent stem cells into the single-cell state, and wherein the pluripotent stem cells in the single-cell state are cultured by the suspension culture in the first liquid medium.
(5) The production method according to any one of (1) to (4), which is characterized by carrying out a step of processing a cell mass collected after the step of performing the suspension culture in the second liquid medium into cells in the single-cell state and performing suspension culture of the cells in the first liquid medium, and repeating the step of performing the suspension culture in the second liquid medium.
(6) The production method according to any one of (1) to (5), which is characterized in that the first liquid medium has a concentration of the PKCβ inhibitor of less than 25 nM.
(7) The production method according to any one of (1) to (6), which is characterized in that the second liquid medium has a concentration of the PKCβ inhibitor of 25 nM or more and 15 µM or less.
(8) The production method according to any one of (1) to (7), which is characterized in that the first liquid medium does not substantially contain the WNT inhibitor or the first liquid medium and/or the second liquid medium has a concentration of the WNT inhibitor of 90 nM or more and 40 µM or less.
(9) The production method according to any one of (1) to (8), which is characterized in that the second liquid medium has a concentration (molar concentration) ratio of the PKCβ inhibitor and the WNT inhibitor is in a range of 167:1 or more and 1:1600 or less.
(10) The production method according to any one of (1) to (9), which is characterized in that the first liquid medium and the second liquid medium contain at least one selected from the group consisting of L-ascorbic acid, insulin, transferrin, selenium, and sodium hydrogen carbonate.
(11) The production method according to any one of (1) to (10), which is characterized in that the first liquid medium and the second liquid medium contain FGF2 and/or TGF-β1.
(12) The production method according to any one of (1) to (11), which is characterized in that the first liquid medium and the second liquid medium contain a ROCK inhibitor.
(13) The production method according to (12), which is characterized in that the ROCK inhibitor is Y-27632.
(14) The production method according to any one of (1) to (13), which is characterized in that the suspension culture is performed by a stirring process in the step performing the suspension culture in the first liquid medium and the step of performing the suspension culture in the second liquid medium, and a blade tip speed of a stirring blade in the step of performing the suspension culture in the second liquid medium is equal to or higher than a blade tip speed of a stirring blade in the step of performing the suspension culture in the first liquid medium.
(15) The production method according to any one of (1) to (14), which is characterized by comprising a step of collecting a cell mass of the pluripotent stem cells after the step of performing the suspension culture in the second liquid medium.
(16) The production method according to any one of (1) to (15), which is characterized in that the step of performing the suspension culture in the first liquid medium is carried out within 48 hours.
(17) The production method according to any one of (1) to (16), which is characterized in that a proportion of cells positive for OCT4 is 90% or more, a proportion of cells positive for SOX2 is 90% or more, and a proportion of cells positive for NANOG is 90% or more, among the pluripotent stem cells.
(18) The production method according to any one of (1) to (17), which is characterized in that the pluripotent stem cells are ES cells and/or induced pluripotent stem cells.
(19) Pluripotent stem cells or a pluripotent stem cell population produced by the production method according to any one of (1) to (18).

In addition, it is preferable to carry out the step of performing the suspension culture in the second liquid medium until a lactic acid concentration in the medium reaches 5 to 15 mM in the method for producing pluripotent stem cells according to the present invention.

Further, it is preferable to perform stirring culture with the blade tip speed of 0.05 m/s or more and 1.37 m/s or less in the step of performing the suspension culture in the first liquid medium and/or the step of performing the suspension culture in the second liquid medium in the method for producing pluripotent stem cells according to the present invention.

Furthermore, the PKCβ inhibitor can be at least one selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo-Dihydrosphingosine, and Melittin in the method for producing pluripotent stem cells according to the present invention.

Moreover, the WNT inhibitor can be at least one selected from the group consisting of IWR-1-endo, XAV939, G007-LK, G244-LM, and WIKI4 which are tankyrase inhibitors (TNKS inhibitors) in the method for producing pluripotent stem cells according to the present invention.

Moreover, primed pluripotent stem cells can be applied as pluripotent stem cells in the method for producing pluripotent stem cells according to the present invention.

Moreover, it is preferable that the above-described first liquid medium and/or the above-described second liquid medium do not contain LIF in the method for producing pluripotent stem cells according to the present invention.

Moreover, it is preferable that the above-described first liquid medium and/or the above-described second liquid medium do not contain a GSK3 inhibitor in the method for producing pluripotent stem cells according to the present invention.

Moreover, it is preferable that the above-described first liquid medium and/or the above-described second liquid medium do not contain a GSK3 inhibitor and a MEK/ERK inhibitor in the method for producing pluripotent stem cells according to the present invention.

Moreover, the PKCβ inhibitor can be a compound represented by the following structural formula [Formula I] in the method for producing pluripotent stem cells according to the present invention.

A compound represented by the following Formula I or a salt thereof:

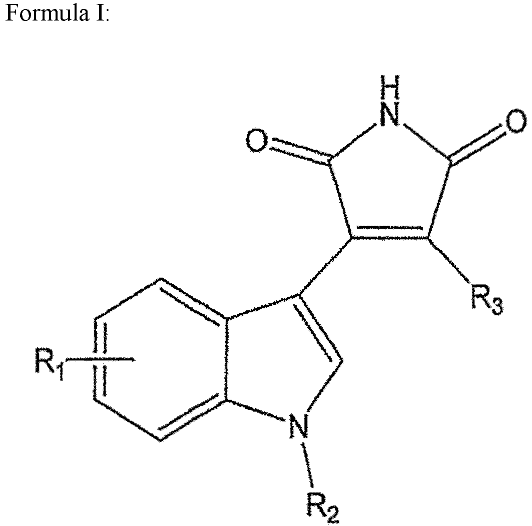

In Formula I,
R₁ is a hydrogen atom or an alkoxy group having 1 to 3 carbon atoms (preferably a methoxy group), R₂ is a hydrogen atom, an alkyl group having 1 to 3 carbon atoms (preferably a methyl group), or a -N(R_{A})₂-substituted alkyl group having 1 to 3 carbon atoms (preferably - (CH₂)₃-N(CH₃)₂),
R_{A} is independently an ethyl group or a methyl group (preferably a methyl group),
R₃ is a group represented by 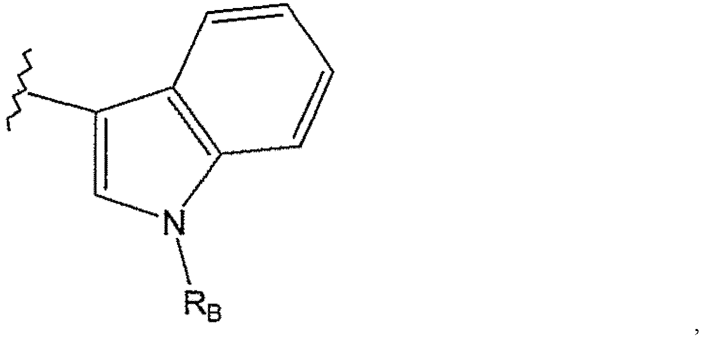 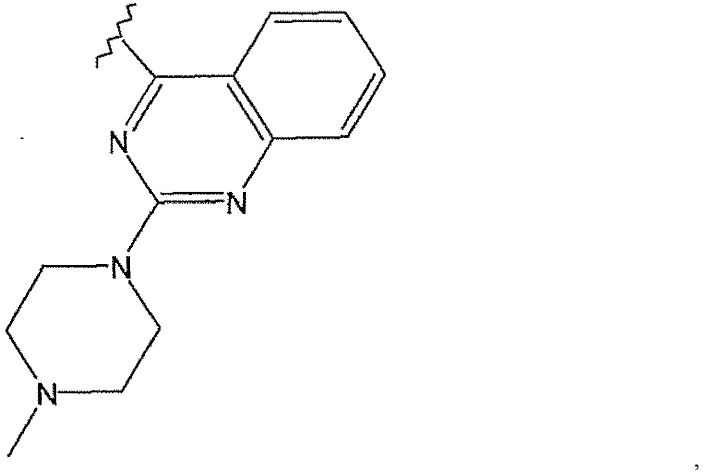 or 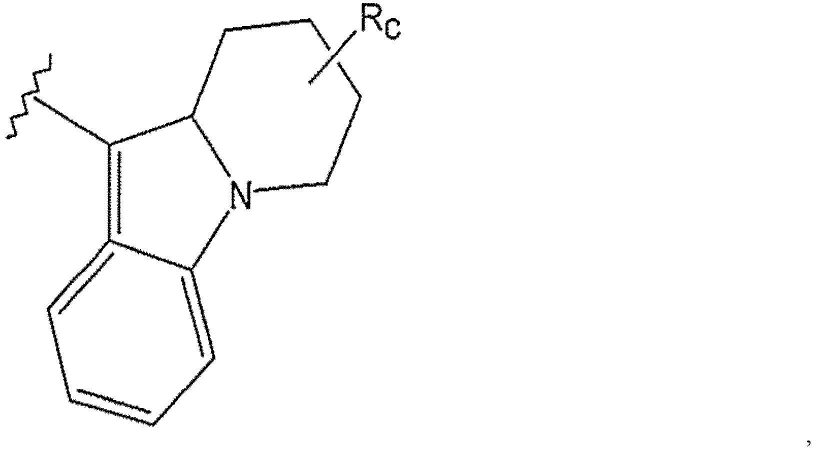
R_{B} is a hydrogen atom, -S-C(=NH)(-NH₂)-substituted alkyl group having 2 to 4 carbon atoms (preferably -(CH₂)₃-S-C(=NH)(-NH₂)), or a group represented by 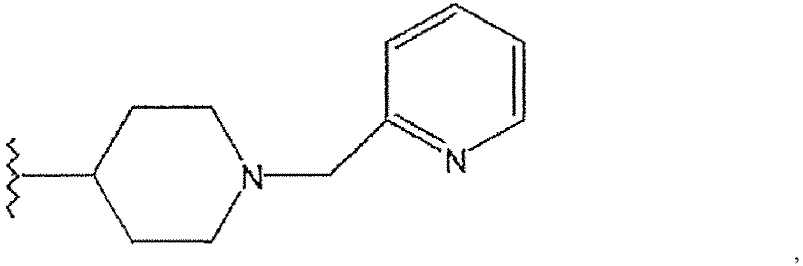
or R₂ and R_{B} may form together the following divalent group: 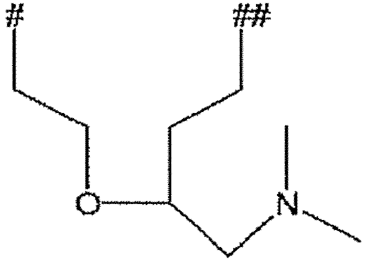 where "#" refers to the bond to the binding position of R₂, "##" refers to the bond to the binding position of R_{B}, and
the steric configuration of the asymmetric carbon contained in the divalent group is not particularly limited, but is preferably 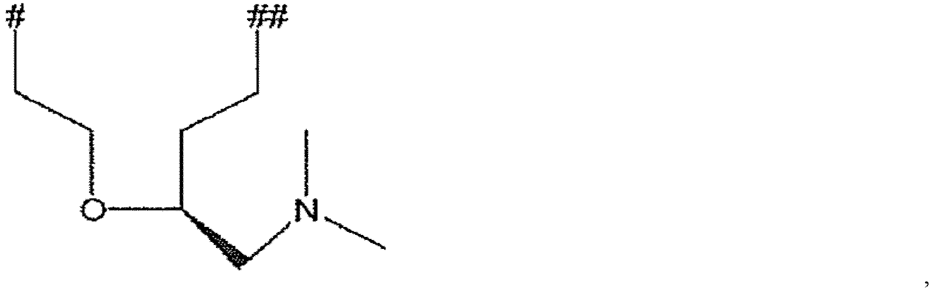
R_{C} is -N(R_{D})₂-substituted alkyl group having 1 to 3 carbon atoms (preferably -(CH₂)-N(CH₃)₂), and
R_{D} is independently an ethyl group or a methyl group (preferably a methyl group).

Examples of the salt of the compound represented by Formula I include hydrochloride and sulfate.

The present specification encompasses the contents described in Japanese Patent Application No. 2021-130903, on which the priority of the present application is based.

### Advantageous Effects of Invention

According to the method for producing pluripotent stem cells according to the present invention, when producing a pluripotent stem cell population by the suspension culture, it is possible not only to obtain high-quality pluripotent stem cells while maintaining an undifferentiated state but also to further achieve excellent cell culture efficiency by preventing cell death.

### Brief Description of Drawings

[Figure 1] Figure 1 is a characteristic diagram showing the expression level of the OCT4 gene (a) and the expression level of the NANOG gene (b) measured by quantitative real-time PCR.
[Figure 2] Figure 2 is a characteristic diagram showing the expression level of the SOX2 gene (a) and the expression level of the TBXT gene (b) measured by quantitative real-time PCR.
[Figure 3] Figure 3 is a characteristic diagram showing the expression level of the SOX17 gene (a) and the expression level of the PAX6 gene (b) measured by quantitative real-time PCR.
[Figure 4] Figure 4 is a characteristic diagram showing the results of counting the number of cells in cell cultures of Reference Examples 6 and 7.
[Figure 5] Figure 5 is a characteristic diagram showing a scheme for adding a PKCβ inhibitor in suspension culture.
[Figure 6] Figure 6 is a characteristic diagram showing the cell density maintenance rate calculated based on the number of cells in suspension culture of Examples 1 and 2, and Comparative Examples 1 and 2.
[Figure 7] Figure 7 is a characteristic diagram showing the results of calculating the specific growth rate of cells cultured by suspension culture in Examples 2 and 3, and Comparative Examples 2 and 3.
[Figure 8] Figure 8 is a characteristic diagram showing the transition in cell density of cells cultured by suspension culture in Examples 2 and 3, and Comparative Examples 2 and 3.
[Figure 9] Figure 9 is a characteristic diagram showing the expression level of the OCT4 gene (a) and the expression level of the NANOG gene (b) measured by quantitative real-time PCR on the cells of Examples 2 and 3, and Comparative Example 2 on days 5 and 10 of culture.
[Figure 10] Figure 10 is a characteristic diagram showing the expression level of the SOX2 gene (a) and the expression level of the TBXT gene (b) measured by quantitative real-time PCR on the cells of Examples 2 and 3, and Comparative Example 2 on days 5 and 10 of culture.
[Figure 11] Figure 11 is a characteristic diagram showing the expression level of the SOX17 gene (a) and the expression level of the PAX6 gene (b) measured by quantitative real-time PCR on the cells of Examples 2 and 3, and Comparative Example 2 on days 5 and 10 of culture.
[Figure 12] Figure 12 is a characteristic diagram showing the positive rate of OCT4 (a), positive rate of SOX2 (b), and positive rate of NANOG (c) measured by dot plot for cells cultured in Examples 2 and 3, and Comparative Example 2.

### Description of Embodiments

### 1. Method for Producing Pluripotent Stem Cells

### 1-1. Outline

The method for producing pluripotent stem cells according to the present invention is characterized by culturing pluripotent stem cells in the presence of a protein kinase Cβ (PKCβ) inhibitor and a WNT inhibitor to suppress the differentiation of the pluripotent stem cells and further performing suspension culture of pluripotent stem cells in the single-cell state in a liquid medium (first liquid medium) that does not substantially contain a PKCβ inhibitor. In particular, the method for producing pluripotent stem cells according to the present invention includes a two-step process of: culturing pluripotent stem cells in a liquid medium (first liquid medium) that does not substantially contain a PKCβ inhibitor until the pluripotent stem cells form a cell mass; and performing suspension culture of the pluripotent stem cells in a liquid medium (second liquid medium) containing a PKCβ inhibitor and a WNT inhibitor after the pluripotent stem cells form the cell mass.

According to the method for producing pluripotent stem cells according to the present invention, pluripotent stem cells or pluripotent stem cell populations that maintain an undifferentiated state can be produced efficiently by preventing cell death of pluripotent stem cells at a stage before forming a cell mass.

### 1-2. Definitions of Terms

The following terms used herein are defined.

### <<Cell>>

The "pluripotent stem cell" that is the subject of the invention herein has multipotency (pluripotency) capable of differentiating into all types of cells that constitute the living body, and it refers to a cell that can continue to grow indefinitely while maintaining pluripotency in *in vitro* culture under the suitable condition. More specifically, pluripotency means the ability to differentiate into germ layers that constitute an individual (corresponding to three germ layers including ectoderm, mesoderm, and endoderm in the case of vertebrates). Examples of such cells can include embryonic stem cells (ES cells), embryonic germ cells (EG cells), germline stem cells (GS cells), and induced pluripotent stem cells (iPS cells). "ES cells" are pluripotent stem cells prepared from early embryos. "EG cells" are pluripotent stem cells prepared from fetal primordial germ cells (Shamblott M. J. et al., 1998, Proc. Natl. Acad. Sci. USA., 95:13726-13731). "GS cells" are pluripotent stem cells prepared from testicular cells (Conrad S., 2008, Nature, 456:344-349). In addition, "iPS cells" refer to pluripotent stem cells reprogrammed to undifferentiated somatic cells by introducing genes encoding a small number of reprogramming factors into differentiated somatic cells.

Pluripotent stem cells as used herein may be cells derived from a multicellular organism. Pluripotent stem cells are preferably animal-derived cells, more preferably mammal-derived cells. Examples thereof include: rodents such as mice, rats, hamsters, and guinea pigs; livestock or pet animals such as dogs, cats, rabbits, bovines, horses, sheep, and goats; and primates such as humans, rhesus monkeys, gorillas, and chimpanzees. Human-derived cells are particularly preferable.

The pluripotent stem cells used herein include naive pluripotent stem cells and primed pluripotent stem cells. Naive pluripotent stem cells are defined as being in a near-pluripotent state found in the pre-implantation inner cell mass. Primed pluripotent stem cells are defined as being in a near-pluripotent state found in the post-implantation epiblast. Primed pluripotent stem cells are characterized in that, compared to naive pluripotent stem cells, they contribute to ontogenesis infrequently, have only one X chromosome which is transcriptionally active state, and have histones modified at a high level for transcriptional repression. In addition, the OTX2 gene is a marker gene for primed pluripotent stem cells, and the REX1 gene and KLF family gene are marker genes for naive pluripotent stem cells. Further, the shape of colonies formed by primed pluripotent stem cells is the flat shape, and the shape of colonies formed by naive pluripotent stem cells is the dome shape. In particular, primed pluripotent stem cells are preferably used as pluripotent stem cells used herein.

The pluripotent stem cells used herein may be commercially available cells, distributed cells, or newly prepared cells. Although not limited, pluripotent stem cells are preferably iPS cells or ES cells when used in each invention described herein.

In a case in which iPS cells used herein are commercially available iPS cells, they are not limited; however, for example, iPS cell lines 253G1, 253G4, 201B6, 201B7, 409B2, 454E2, 606A1, 610B1, 648A1, HiPS-RIKEN-1A, HiPS-RIKEN-2A, HiPS-RIKEN-12A, Nips-B2, TkDN4-M, TkDA3-1, TkDA3-2, TkDA3-4, TkDA3-5, TkDA3-9, TkDA3-20, hiPSC38-2, MSC-iPSC1, BJ-iPSC1, RPChiPS771-2, WTC-11, 1231A3, 1383D2, 1383D6, 1210B2, 1201C1, and 1205B2 can be used.

In addition, in a case in which the iPS cell line used herein is a clinical cell line, examples of the clinical cell line that can be used include, but are not limited to, QHJI01s01, QHJI01s04, QHJI14s03, QHJI14s04, Ff-l14s03, Ff-l14s04, and YZWI.

In a case in which the iPS cells used herein are newly prepared cells, examples of a combination of genes of reprogramming factors to be introduced, which can be used, include, but are not limited to, a combination of the OCT3/4 gene, the KLF4 gene, the SOX2 gene, and the c-Myc gene (Yu J, et al. 2007, Science, 318:1917-20) and a combination of the OCT3/4 gene, the SOX2 gene, the LIN28 gene, and the NANOG gene (Takahashi K, et al. 2007, Cell, 131:861-72). The form of introducing these genes into cells is not particularly limited; however, it may be, for example, the gene introduction using a plasmid, introduction of synthetic RNA, or introduction of a gene as a protein. In addition, iPS cells produced by a method using microRNA, RNA, a low-molecular-weight compound, or the like may also be used. Needless to say, newly prepared clinical grade iPS cells may be used.

In a case in which ES cells used herein are commercially available ES cells, they are not limited; however, for example, ES cell lines KhES-1, KhES-2, KhES-3, KhES-4, KhES-5, SEES1, SEES2, SEES3, SEES-4, SEES-5, SEES-6, SEES-7, HUES8, CyT49, H1, H9, and HS-181 can be used.

### <<Cell Mass>>

As used herein, the "cell mass" refers to a massive aggregate formed by cell aggregation in suspension culture and is also called a spheroid. A cell mass is usually roughly spherical. Cells constituting a cell mass are not particularly limited as long as they are of one or more types of the cells described above. For example, a cell mass composed of pluripotent stem cells such as human pluripotent stem cells or human embryonic stem cells comprises cells expressing pluripotent stem cell markers and/or positive for pluripotent stem cell markers. In addition, the cell mass may be formed via microcarriers.

Pluripotent stem cell markers are gene markers that are specifically or excessively expressed in pluripotent stem cells. Examples thereof can include Alkaline Phosphatase, NANOG, OCT4, SOX2, TRA-1-60, c-Myc, KLF4, LIN28, SSEA-4, and SSEA-1.

Pluripotent stem cell markers can be detected by any detection method in the art. Examples of a method for detecting a cell marker include, but are not limited to, flow cytometry. For example, in the case of using a fluorescence-labeled antibody as the detection reagent in flow cytometry, when cells emitting stronger fluorescence than the negative control (isotype control or FMO control) are detected, the cells can be determined to be "positive" for the marker. The percentage of cells positive for a fluorescence-labeled antibody as analyzed by flow cytometry is sometimes referred to as the "positive rate." Any antibody known in the art can be used as a fluorescence-labeled antibody. Examples thereof include, but are not limited to, antibodies labeled with fluorescein isothiocyanate (FITC), phycoerythrin (PE), and allophycocyanin (APC).

In a case in which cells constituting a cell mass are pluripotent stem cells, the positive rate of the pluripotent stem cell marker is preferably 80% or more, more preferably 90% or more, still more preferably 91% or more, even more preferably 92% or more, yet more preferably 93% or more, yet even more preferably 94% or more, yet even more preferably 95% or more, yet even more preferably 96% or more, yet even more preferably 97% or more, yet even more preferably 98% or more, yet even more preferably 99% or more, yet even more preferably 100%. A cell mass in which the proportion of cells expressing a pluripotent stem cell marker and/or being positive for a pluripotent stem cell marker is within the above-described numerical range is a highly undifferentiated and more homogeneous cell aggregate.

### <<Culture and Medium>>

"Suspension culture" is one of the cell culture methods and refers to allowing cells to grow in a suspension state in a medium. As used herein, the "suspension state" refers to a non-adherent state in which cells are not fixed to a culture vessel or the like in a culture solution. In addition, for example, a culture method in which cells which are adhered to microcarriers are cultured while suspended in a culture solution can be regarded as the suspension culture. Because a cell mass containing the microcarriers floats without adhering to the culture vessel although the cells adhere to the microcarriers. The "suspension culture method" is a method of culturing cells by the suspension culture. Cells in this method generally exist primarily in a single-cell state at the start of the culture, and as the culture progresses, the cells exist in cell masses aggregated in the culture solution. An alternative culture method to the suspension culture method is the adherent culture method. The "adherent culture method" is a method of adherent culture of cells. "Adherent culture" refers to allowing cells to grow by allowing the cells to adhere an external matrix such as a culture vessel. At such time, the external matrix and the like to which the cells have adhered are not suspended in the culture solution. In general, the adherent cells described above can be cultured not only by the adherent culture but also by the suspension culture.

The terms "first liquid medium" and "second liquid medium" are used herein. The first liquid medium and the second liquid medium differ in that the former does not substantially contain a PKCβ inhibitor, but the latter contains a PKCβ inhibitor. Note that although a WNT inhibitor is always contained in the second liquid medium, the WNT inhibitor may or may not be contained in the first liquid medium.

As used herein, the "medium" refers to a liquid or solid substance prepared for culturing cells. In principle, a medium contains at least the minimum necessary amount of components essential for cell growth and/or maintenance. Unless otherwise specified, the medium herein corresponds to a liquid medium for animal cells used for culturing animal-derived cells.

As used herein, the "basal medium" refers to a medium that is the basis for various animal cell culture media. Culture is possible with a basal medium alone. It is also possible to prepare a medium according to the purpose, for example, a medium specific to various cells, by adding various culture additives. Examples of a basal medium that can be used herein include, but are not particularly limited to, BME medium, BGJb medium, CMRL1066 medium, Glasgow MEM medium, Improved MEM Zinc Option medium, Iscove's Modified Dulbecco's Medium (IMDM medium), Medium 199, Eagle MEM medium, α MEM medium, Dulbecco's Modified Eagle's Medium (DMEM medium), Ham's F10 medium, Ham's F12 medium, RPMI 1640 medium, Fischer's medium, and mixed medium thereof (e.g., DMEM/F12 medium (Dulbecco's Modified Eagle's Medium/Nutrient Mixture F-12 Ham)). As DMEM/F12 medium, in particular, a medium obtained by mixing DMEM medium and Ham's F12 medium at a weight ratio in a range of preferably 60/40 to 40/60, for example, 58/42, 55/45, 52/48, 50/50, 48/52, 45/55, or 42/58 is used. Other media such as those used for culturing human iPS cells and human ES cells can also be appropriately used.

Media for use in the present invention are preferably media that do not contain serum, i.e., serum-free media.

As used herein, a "culture additive" is a substance other than serum added to a medium for culture. Specific examples of a culture additive include, but are not limited to, L-ascorbic acid, insulin, transferrin, selenium, sodium hydrogen carbonate, growth factors, fatty acids or lipids, amino acids (e.g., non-essential amino acids), vitamins, cytokines, antioxidants, 2-mercaptoethanol, pyruvic acid, buffers, inorganic salts, and antibiotics. Insulin, transferrin, and cytokines may be naturally occurring proteins isolated from tissues or serum of animals (preferably humans, mice, rats, bovines, horses, goats, and the like) or may be genetically engineered recombinant proteins. Examples of growth factors include, but are not limited to, basic fibroblast growth factor-2 (FGF2), transforming growth factor-β1 (TGF-β1), Activin A, IGF-1, MCP-1, IL-6, PAI, PEDF, IGFBP-2, LIF, and IGFBP-7. Examples of antibiotics that can be used include, but are not limited to, penicillin, streptomycin, and amphotericin B. Particularly preferable growth factors as culture additives for the medium used in the present invention are FGF2 and/or TGF-β1.

In addition, preferably, the medium contains a ROCK inhibitor. Examples of a ROCK inhibitor include Y-27632. By including a ROCK inhibitor in the medium, cell death in the suspension culture of pluripotent stem cells can be significantly suppressed, and the strength of the cell mass can be increased such that resistance to physical damage can be improved. For example, the concentration of the ROCK inhibitor in the medium can have a lower limit of 1 µM, 2 µM, 3 µM, 5 µM, 7 µM, or 10 µM, and an upper limit of 50 µM, 40 µM, 30 µM, 20 µM, or 10 µM.

Further, the medium preferably has a composition that does not contain LIF, especially in a case in which primed pluripotent stem cells are to be cultured. Furthermore, it is preferable that the medium composition does not contain either one of, preferably both of a GSK3 inhibitor and a MEK/ERK inhibitor in a case in which primed pluripotent stem cells are to be cultured. It is possible to culture primed pluripotent stem cells while maintaining their undifferentiated state without making the primed pluripotent stem cells naive in a medium that does not contain LIF, a GSK3 inhibitor, and a MEK/ERK inhibitor.

The medium used in the present invention may contain one or more culture additives described above. Examples of a medium to be supplemented with the culture additives include, but are not limited to. In general, the medium is the basal medium described above.

A culture additive can be added to the medium in the form of a solution, a derivative, a salt, a mixed reagent, or the like. For example, L-ascorbic acid may be added to the medium in the form of a derivative such as magnesium ascorbate 2-phosphate. Selenium may be added to the medium in the form of a selenite salt (e.g., sodium selenite). In addition, insulin, transferrin, and selenium can also be added to the medium in the form of an insulin-transferrin-selenium (ITS) reagent. A commercially available medium supplemented with at least one selected from L-ascorbic acid, insulin, transferrin, selenium, or sodium hydrogen carbonate can also be used. Examples of commercially available media supplemented with insulin and transferrin include CHO-S-SFM II (Life Technologies Japan Ltd.), Hybridoma-SFM (Life Technologies Japan Ltd.), eRDF Dry Powdered Media (Life Technologies Japan Ltd.), UltraCULTURE (trademark) (BioWhittaker), UltraDOMA (trademark) (BioWhittaker), UltraCHO (trademark) (BioWhittaker), UltraMDCK (trademark) (BioWhittaker), STEMPRO (registered trademark) hESC SFM (Life Technologies Japan Ltd.), Essentials (trademark) (Life Technologies Japan Ltd.), StemFit (registered trademark) AK02N (AJINOMOTO CO., INC.), mTeSR1 (Veritas), TeSR2 (Veritas), ReproMed (REPROCELL Inc.), and StemScale (Thermo Fisher Scientific Inc.).

Medium that is most preferably used in the present invention is a serum-free medium containing L-ascorbic acid, insulin, transferrin, selenium, and sodium hydrogen carbonate and at least one growth factor. A serum-free DMEM/F12 medium containing L-ascorbic acid, insulin, transferrin, selenium and sodium hydrogen carbonate, and at least one growth factor (preferably FGF2 and TGF-β1) are particularly preferable.

### <<PKCβ Inhibitor>>

As used herein, "protein kinase Cβ (PKCβ) inhibitor" refers to a substance that inhibits or suppresses the activity of PKCβ. Protein kinases have a C-terminal catalytic region and an N-terminal regulatory region. The catalytic region is composed of a sequence that recognizes a phosphorylated residue on a substrate protein and a sequence that forms an ATP-Mg²⁺ binding site. The regulatory region is composed of C1 domain and C2 domain.

PKC includes PKCα, PKCβI, PKCβII, and PKCγ as conventional isozymes. Further, PKC includes PKC6, PKCε, PKCθ, and PKCη as novel isozymes, and PKCζ, PKCλ, and PKCµ as atypical isozymes.

As used herein, PKCβ refers to a case of including both PKCβI and PKCβII or a case of including one of PKCβI and PKCβII. As used herein, the "PKCβ inhibitor" refers to a substance that inhibits at least PKCβI and/or PKCβII among these conventional, novel, and atypical isozymes. In other words, the PKCβ inhibitor includes a substance that inhibits or suppresses only the activity of PKCβI, a substance that inhibits or suppresses only the activity of PKCPII, and a substance that inhibits or suppresses the activities of PKCPI and PKCβII.

The PKCβ inhibitor may be a substance that specifically inhibits or suppresses only the activity of PKCβ, but may also be a substance that inhibits or suppresses the activities of other isozymes other than PKCβI or PKCβII. For example, the PKCβ inhibitor may be a substance that inhibits or suppresses the activities of all the conventional, novel, and atypical isozymes described above, including PKCβI and PKCβII. In addition, the PKCβ inhibitor may be a substance that inhibits or suppresses the activities of conventional isozymes PKCα, and PKCγ in addition to PKCβI and PKCβII. Further, the PKCβ inhibitor may be a substance that inhibits or suppresses the activities of novel isoenzymes PKCδ, PKCε, PKCθ, and PKCη in addition to PKCβI and PKCβII.

The above-described PKCβ inhibitors include compounds that act directly or indirectly on PKCβ, antisense nucleic acids against the gene encoding PKCβ, RNA interference-inducing nucleic acids (e.g., siRNA), dominant negative mutants, and expression vectors thereof.

As an example, the PKCβ inhibitor can include a compound having the following structural formula [Formula I].

A compound represented by the following Formula I or a salt thereof:

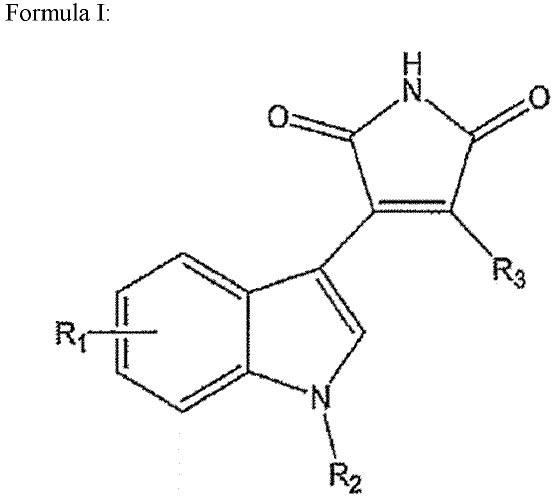

In Formula I,
R₁ is a hydrogen atom or an alkoxy group having 1 to 3 carbon atoms (preferably a methoxy group), R₂ is a hydrogen atom, an alkyl group having 1 to 3 carbon atoms (preferably a methyl group), or a -N(R_{A})₂-substituted alkyl group having 1 to 3 carbon atoms (preferably - (CH₂)₃-N(CH₃)₂),
R_{A} is independently an ethyl group or a methyl group (preferably a methyl group),
R₃ is a group represented by 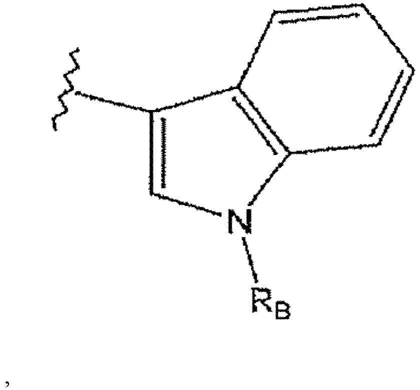 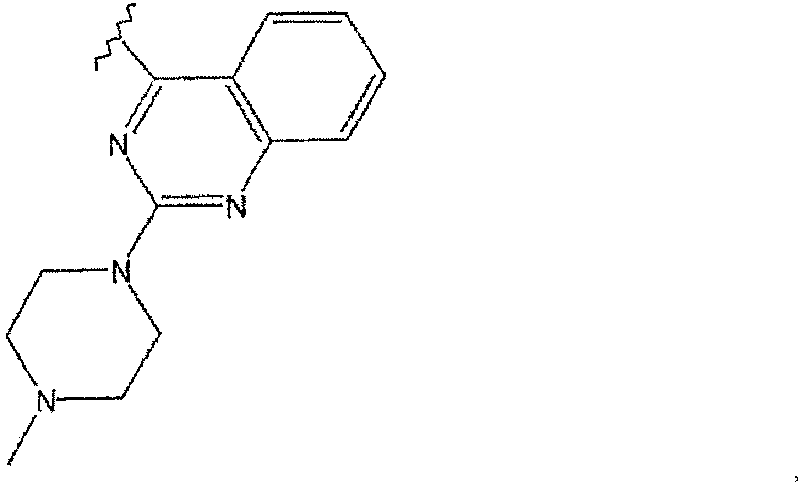 or 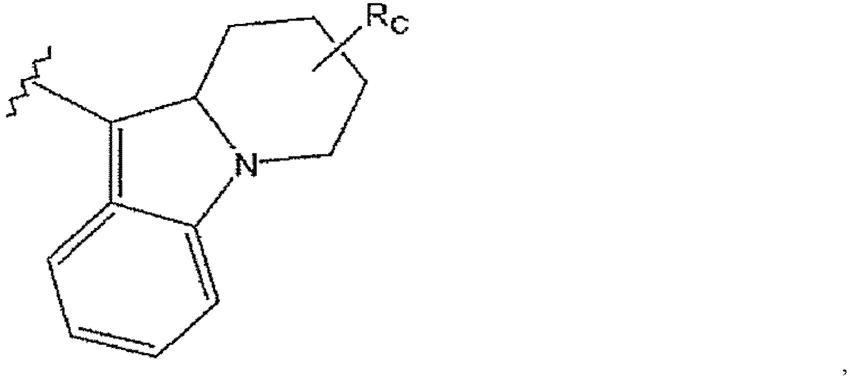
R_{B} is a hydrogen atom, -S-C(=NH)(-NH₂)-substituted alkyl group having 2 to 4 carbon atoms (preferably -(CH₂)₃-S-C(=NH)(-NH₂)), or
a group represented by 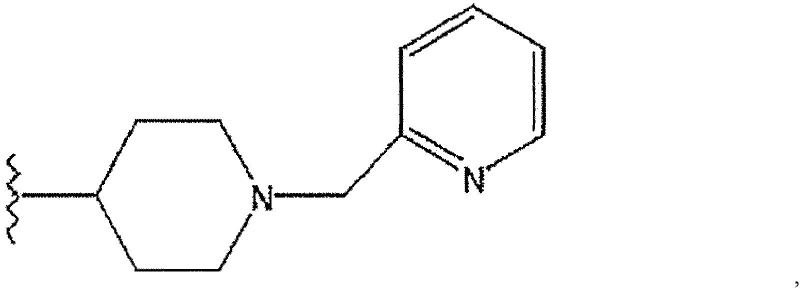
or R₂ and R_{B} may form together the following divalent group: 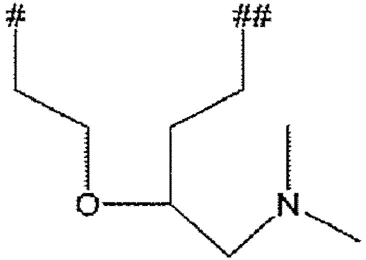 where "#" refers to the bond to the binding position of R₂, "##" refers to the bond to the binding position of R_{B}, and
the steric configuration of the asymmetric carbon contained in the divalent group is not particularly limited, but is preferably 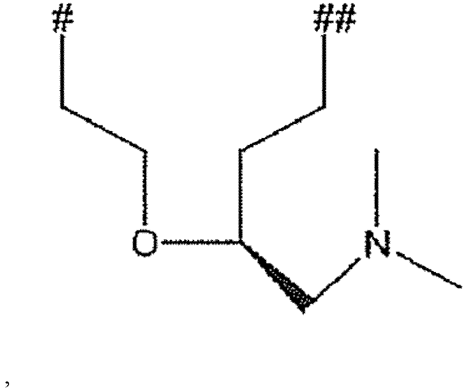
R_{C} is -N(R_{D})₂-substituted alkyl group having 1 to 3 carbon atoms (preferably -(CH₂)-N(CH₃)₂), and
R_{D} is independently an ethyl group or a methyl group (preferably a methyl group).

Examples of the salt of the compound represented by Formula I include hydrochloride and sulfate.

Specific examples of the PKCβ inhibitor with the above-described structural formula [Formula I] can include compounds selected from the group consisting of Go6983, GF109203X, LY-333531, Enzastaurin, Sotrastaurin, Ro-31-8220-mesylate, Ro-32-0432-hydrochloride, Go6976, Rottlerin, Midostaurin, Daphnetin, Dequalinium Chloride, Baicalein, Quercetin, Luteolin, Bisindolylmaleimide II, Calphostin C, Chelerythrine chloride, L-threo-Dihydrosphingosine, and Melittin. It is particularly preferable to use compounds selected from the group consisting of Go6983, GF109203X, and LY-333531 among PKCβ inhibitors with the above-described structural formula.

The structural formula of Go6983 (3-[1-[3-(dimethylamino)propyl]-5-methoxy-1H-indole-3-yl]-4-(1H-indole-3-yl)-1H-pyrrole-2,5-dione) is shown below.

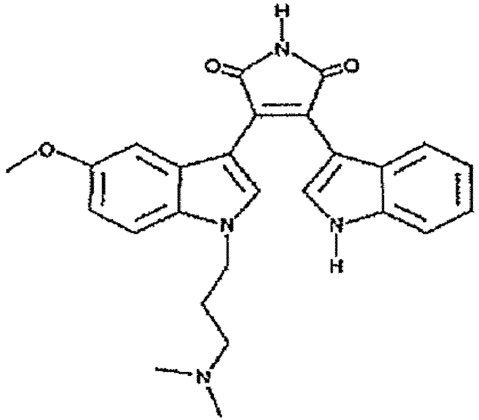

The structural formula of GF109203X (2-[1-(3-dimethylaminopropyl)indole-3-yl]-3-(indole-3-yl)maleimide) is shown below.

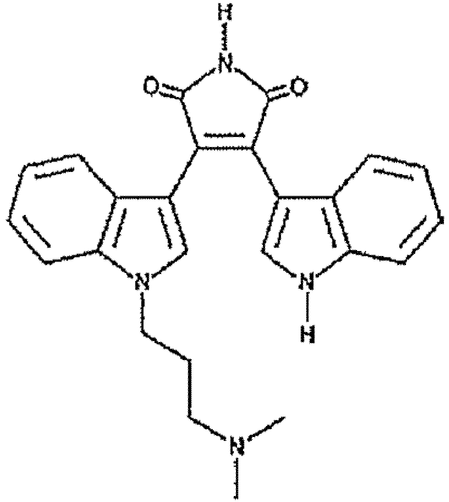

The structural formula of LY-333531((9S)-[(dimethylamino)methyl]-6,7,10,11-tetrahydro-9H,18H-5,21:12,17-dimethenodibenzo[e,k]pyrrolo[3,4-h][1,4,13]oxadiazacyclohexadecine-18,20(19H)-dione, monohydroxy chloride) is shown below.

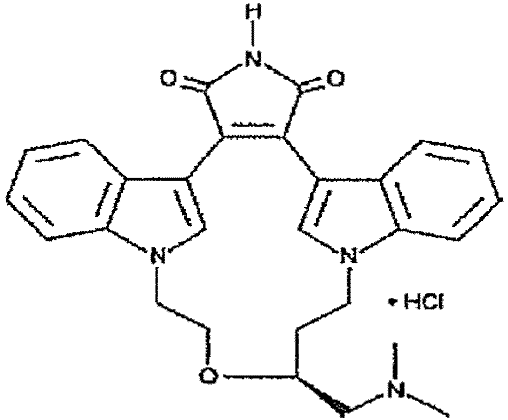

The structural formula of Enzastaurin (3-(1-methylindole-3-yl)-4-[1-[1-(pyridine-2-ylmethyl) piperidine-4-yl]indole-3-yl]pyrrole-2,5-dione) is shown below.

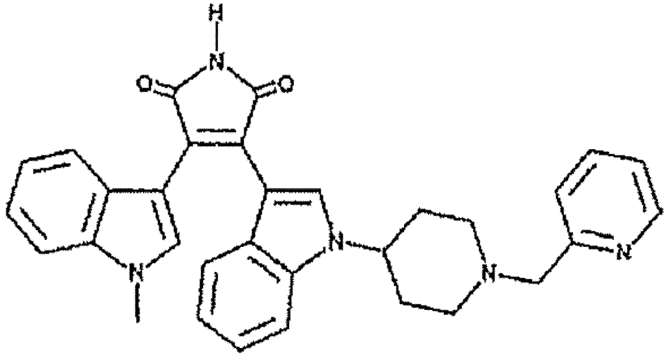

The structural formula of Sotrastaurin (3-(1H-indole-3-yl)-4-(2-(4-methylpiperazine-1-yl)quinazoline-4-yl)-1H-pyrrole-2,5-dione) is shown below.

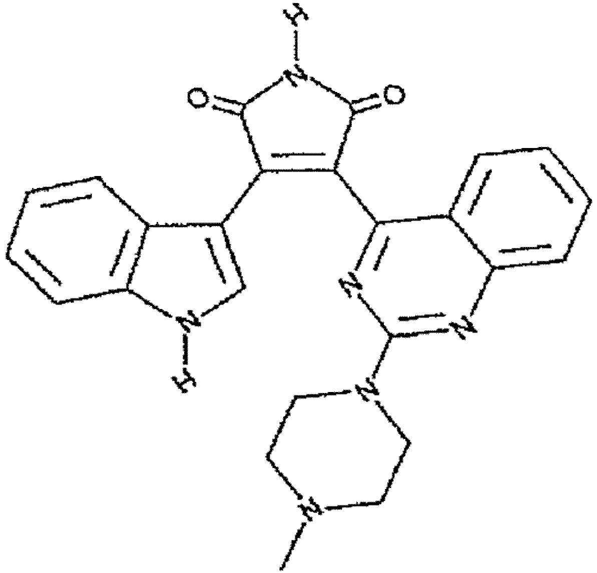

The structural formula of Ro-31-8220-mesylate (3-[3-[2,5-dihydro-4-(1-methyl-1H-indole-3-yl)-2,5-dioxo-1H-pyrrole-3-yl]-1H-indole-1-yl]propyl carbamimidic thio acid ester mesylate) is shown below.

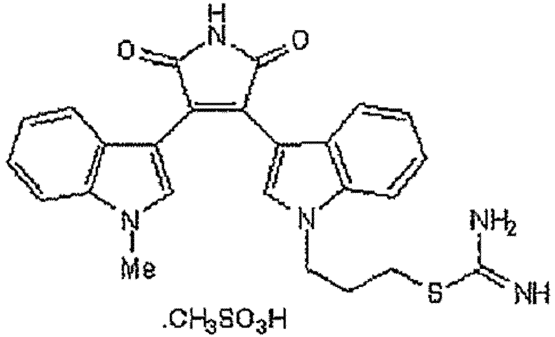

The structural formula of Ro-32-0432-hydrochloride (3-[(8S)-8-[(dimethylamino)methyl]-6,7,8,9-tetrahydropyrido[1,2-a]indole-10-yl]-4-(1-methyl-1H-indole-3-yl)-1H-pyrrole-2,5-dione hydrochloride) is shown below.

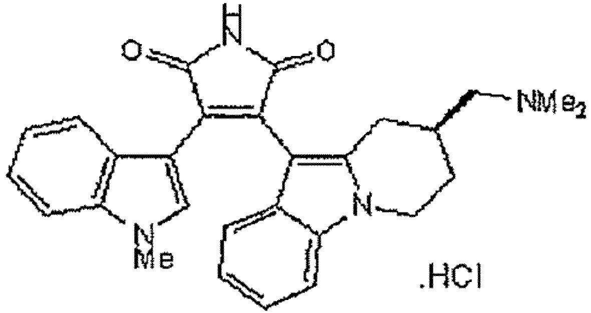

### <<WNT Inhibitor>>

As used herein, the "WNT signal (WNT) inhibitor" includes all substances that inhibit or reduce Wnt signals. Wnt is a secretory intercellular signal transduction protein and is known to be involved in intracellular signal transduction. The signal transduction pathway involving Wnt controls functions such as cell growth, differentiation, and movement, as well as body axis formation and organ formation during early embryonic development. The signal pathway involving Wnt includes several different intracellular signal transduction mechanisms that are activated by Wnt acting on cells. The signal pathway involving Wnt includes the β-catenin pathway that controls gene expression via β-catenin, the planar cell polarity (PCP) pathway that controls the planar cell polarity of cells, and the Ca²⁺ pathway that promotes intracellular Ca²⁺ mobilization. As used herein, the WNT inhibitor may inhibit any pathway included in the signal transduction pathway involving Wnt.

Examples of the WNT inhibitor include proteins such as secretory Frizzled-related protein, Dickkopf (DKK) protein, Sclerostin, Cerberus, and Kremen. Specific examples of the WNT inhibitor include, but are not particularly limited to, IWR-1-endo, IWP-2, IWP-3, WNT-C59, XAV-939, CCT251545, KY1220, iCRT14, iCRT3, LF3, PNU-74654, KYA1797K, Capmatinib (INCB28060), KY02111, RCM-1, Resibufogenin, MSAB, PRI-724, Tegatrabetan (BC-2059), JW55, Adavivint (SM04690), Triptonide, Isoquercitrin, IQ-1, Salinomycin, and FH535. Further, examples of the WNT inhibitor include Wnt receptor inhibitors, soluble Wnt receptors, Wnt antibodies, casein kinase inhibitors, and dominant-negative Wnt proteins.

These WNT inhibitors include so-called tankyrase (TNKS) inhibitors. In the present invention, it is particularly preferable to use a TNKS inhibitor as the WNT inhibitor.

### <<TNKS Inhibitor>>

As used herein, the " tankyrase (TNKS) inhibitor" refers to a substance that inhibits or suppresses the activity of tankyrase. Tankyrase belongs to the poly (ADP-ribosyl) polymerase (PARP) family, which poly(ADP-ribosyl)ates target proteins. Tankyrase-1 (tankyrase-1/PARP-5a) and tankyrase-2 (tankyrase-2/PARP-5b) are known. Tankyrase is known to have the function of promoting telomere elongation due to telomerase by converting the telomere protein TRF1 into poly (ADP-ribosyl) and releasing it from telomere.

As used herein, TNKS refers to a case of including both tankyrase 1 and tankyrase 2 or a case of including one of tankyrase 1 and tankyrase 2. As used herein, the TNKS inhibitor refers to a substance that inhibits tankyrase 1 and/or tankyrase 2. In other words, the TNKS inhibitor includes a substance that inhibits or suppresses only the activity of tankyrase 1, a substance that inhibits or suppresses only the activity of tankyrase 2, and a substance that inhibits or suppresses the activities of tankyrase 1 and tankyrase 2.

Examples of the TNKS inhibitor include compounds that act directly or indirectly on TNKS, antisense nucleic acids against the gene encoding TNKS, RNA interference-inducing nucleic acids (e.g., siRNA), dominant negative mutants, and expression vectors thereof.

As an example, the TNKS inhibitor can be a compound selected from the group consisting of IWR-1-endo, XAV939, G007-LK, G244-LM, MSC2504877, and WIKI4. It is particularly preferable to use IWR-1-endo and/or XAV939 among TNKS inhibitors.

The structural formula of IWR-1-endo (4-(1,3,3a,4,7,7a-hexahydro-1,3-dioxo-4,7-methano-2H-isoindole-2-yl)-N-8-quinolinyl-benzamide) is shown below.

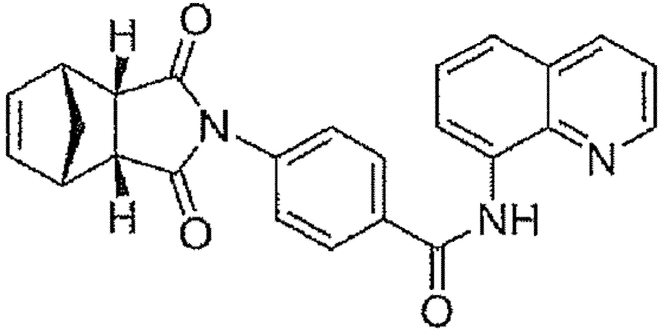

The structural formula of XAV939 (3,5,7,8-tetrahydro-2-[4-(trifluoromethyl) phenyl]-4H-thiopyrano[4,3-d]pyrimidine-4-one) is shown below.

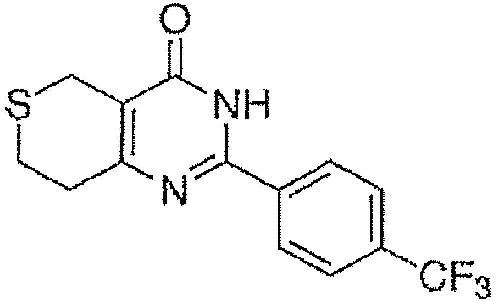

The structural formula of G007-LK (4-[5-[(1E)-2-[4-(2-chlorophenyl)-5-[5-(methylsulfonyl)-2-pyridinyl]-4H-1,2,4-thiazol-3-yl]ethenyl]-1,3,4-oxadiazole-2-yl]-benzonitrile) is shown below.

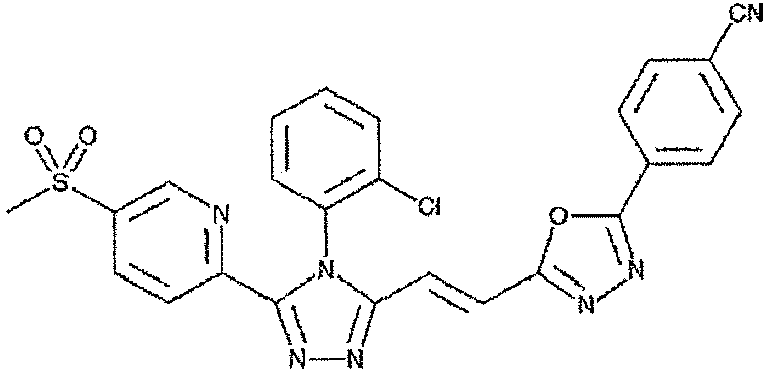

The structural formula of G244-LM (3,5,7,8-tetrahydro-2-[4-[2-(methylsulfonyl) phenyl]-1-piperazinyl]-4H-thiopyrano[4,3-d]pyrimidine-4-one) is shown below.

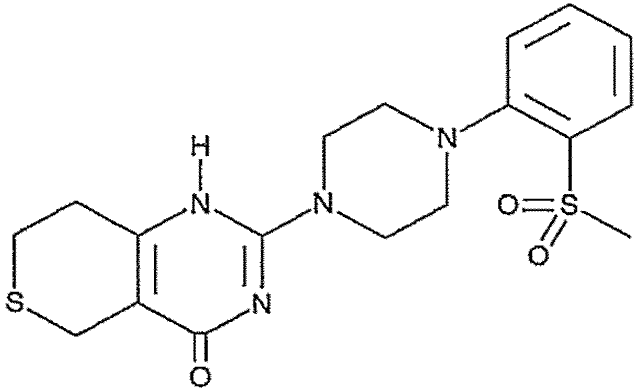

The structural formula of WIKI4 (2-[3-[[4-(4-methoxyphenyl)-5-(4-pyridinyl)-4H-1,2,4-thiazol-3-yl]thio] propyl]-1H-benzo[de]isoquinoline-1,3(2H)-dione) is shown below.

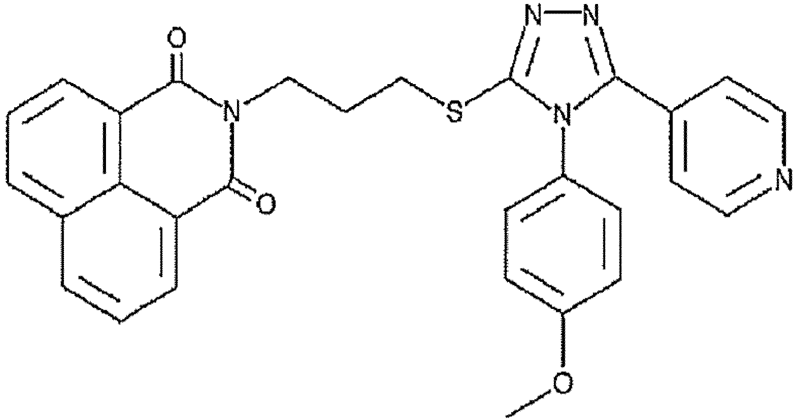

### 1-3. Configuration

A second liquid medium used in the method for producing pluripotent stem cells according to the present invention contains a PKCβ inhibitor and a WNT inhibitor as defined in 1-2, above. Note that the second liquid medium can also be prepared using a pluripotent stem cell differentiation inhibitor containing a PKCβ inhibitor and a WNT inhibitor as active ingredients.

Here, the second liquid medium may contain one type of PKCβ inhibitor or a combination of two or more different types.

The lower limit of the concentration of the PKCβ inhibitor is not particularly limited and can be determined depending on the range in which the PKCβ inhibitor has a differentiation-inhibiting effect on pluripotent stem cells.

For example, the final concentration of the PKCβ inhibitor in the second liquid medium can be 25 nM or more, 30 nM or more, 50 nM or more, 80 nM or more, 100 nM or more, 150 nM or more, 200 nM or more, 500 nM or more, or 700 nM or more.

The upper limit of the concentration of the PKCβ inhibitor is not particularly limited and can be determined depending on the range in which the PKCβ inhibitor has a differentiation-suppressing effect on pluripotent stem cells, the range in which the PKCβ inhibitor does not exhibit toxicity to pluripotent stem cells, the solubility of the PKCβ inhibitor, and the like.

For example, the final concentration of the PKCβ inhibitor in the second liquid medium can be 15 µM or less, 10 µM or less, 5 µM or less, 3 µM or less, or 1 µM or less.

Meanwhile, the second liquid medium may contain one type of WNT inhibitor or a combination of two or more different types.

The lower limit of the concentration of the WNT inhibitor is not particularly limited and can be determined depending on the range in which the WNT inhibitor has a differentiation-inhibiting effect on pluripotent stem cells.

For example, the final concentration of the WNT inhibitor in the second liquid medium can be 90 nM or more, 100 nM or more, 150 nM or more, 200 nM or more, 300 nM or more, 400 nM or more, 500 nM or more, 600 nM or more, 700 nM or more, 800 nM or more, or 900 nM or more.

The upper limit of the concentration of the WNT inhibitor is not particularly limited and can be determined depending on the range in which the WNT inhibitor has a differentiation-suppressing effect on pluripotent stem cells, the range in which the WNT inhibitor does not exhibit toxicity to pluripotent stem cells, the solubility of the WNT inhibitor, and the like.

For example, the final concentration of the WNT inhibitor in the second liquid medium can be 40 µM or less, 35 µM or less, 30 µM or less, 15 µM or less, 10 µM or less, 5 µM or less, 3 µM or less, 1.5 µM or less, or 1 µM or less.

Further, the lower limit of the concentration (molar concentration) ratio of the PKCβ inhibitor and the WNT inhibitor in the second liquid medium is not particularly limited but can be, for example, 167:1 or more, 111:1 or more, 56:1 or more, 33:1 or more, 11:1 or more, 7.8:1 or more, 5.6:1 or more, 2.2:1 or more, 1.7 :1 or more, or 1.1:1 or more. The upper limit of the concentration (molar concentration) ratio of the PKCβ inhibitor and the WNT inhibitor in the second liquid medium is not particularly limited but can be, for example, 1:1600 or less, 1:1400 or less, 1:1200 or less, 1:600 or less, 1:400 or less, 1:200 or less, 1:120 or less, 1:60 or less, 1:40 or less, 1:36 or less, 1:32 or less, 1:28 or less, 1:24 or less, 1:20 or less, 1:16 or less, 1:12 or less, 1:8 or less, 1:6 or less, or 1:4 or less. The concentration (molar concentration) ratio of the PKCβ inhibitor and the WNT inhibitor in the second liquid medium is not particularly limited but can be, for example, in a range of 167:1 or more and 1:1600 or less. In addition, the concentration (molar concentration) ratio of the PKCβ inhibitor and the WNT inhibitor in the second liquid medium can be in a range of preferably 1 11:1 or more and 1:1600 or less, 56:1 or more and 1:1600 or less, 33:1 or more and 1:1600 or less, 11:1 or more and 1:1600 or less, 7.8:1 or more and 1:1600 or less, 5.6:1 or more and 1:1600 or less, 2.2:1 or more and 1:1600 or less, 1.7:1 or more and 1:1600 or less, or 1.1:1 or more and 1:1600 or less. Further, the concentration (molar concentration) ratio of the PKCβ inhibitor and the WNT inhibitor in the second liquid medium can be in a range of 167:1 or more and 1:1400 or less, 167:1 or more and 1:1200 or less, 167:1 or more and 1:600 or less, 167:1 or more and 1:400 or less, 167:1 or more and 1:200 or less, 167:1 or more and 1:120 or less, 167:1 or more and 1:60 or less, 167:1 or more and 1:40 or less, 167:1 or more and 1:36 or less, 167:1 or more and 1:32 or less, 167:1 or more and 1:28 or less, 167:1 or more and 1:24 or less, 167:1 or more and 1:20 or less, 167:1 or more and 1:16 or less, 167:1 or more and 1:12 or less, 167:1 or more and 1:8 or less, 167:1 or more and 1:6 or less, or 167:1 or more and 1:4 or less.

In addition, the PKCβ inhibitor and the WNT inhibitor as active ingredients can also be added to the medium in combination with a carrier. Carriers used herein include solvents and/or excipients.

Examples of solvents include water, buffers (including PBS), physiological saline, and organic solvents (DMSO, DMF, xylene, and lower alcohols).

Examples of excipients can include antibiotics, buffers, thickeners, colorants, stabilizers, surfactants, emulsifiers, antiseptics, preservatives, and antioxidants. Examples of antibiotics that can be used include, but are not particularly limited to, penicillin, streptomycin, and amphotericin B. Examples of buffers include phosphate buffer, Tris-HCl buffer, and glycine buffer. Examples of thickeners include gelatin and polysaccharides. Examples of colorants include phenol red. Examples of stabilizers include albumin, dextran, methyl cellulose, and gelatin. Examples of surfactants include cholesterol, alkyl glycoside, alkyl polyglucoside, alkyl monoglyceryl ether, glucoside, maltoside, neopentyl glycol type, polyoxyethylene glycol type, thioglucoside, thiomaltoside, peptide, saponin, phospholipid, fatty acid sorbitan ester, and fatty acid diethanolamide. Examples of emulsifiers include glycerin fatty acid ester, sorbitan fatty acid ester, propylene glycol fatty acid ester, and sucrose fatty acid ester. Examples of antiseptics include aminoethyl sulfonic acid, benzoic acid, sodium benzoate, ethanol, sodium edetate, agar, dl-camphor, citric acid, sodium citrate, salicylic acid, sodium salicylate, phenyl salicylate, dibutylhydroxytoluene, sorbic acid, potassium sorbate, nitrogen, dehydroacetic acid, sodium dehydroacetate, 2-naphthol, white sugar, honey, isobutyl paraoxybenzoate, isopropyl paraoxybenzoate, ethyl paraoxybenzoate, butyl paraoxybenzoate, propyl paraoxybenzoate, methyl paraoxybenzoate, l-menthol, and eucalyptus oil. Examples of preservatives include benzoic acid, sodium benzoate, ethanol, sodium edetate, dry sodium sulfite, citric acid, glycerin, salicylic acid, sodium salicylate, dibutylhydroxytoluene, D-sorbitol, sorbic acid, potassium sorbate, sodium dehydroacetate, isobutyl paraoxybenzoate, isopropyl paraoxybenzoate, ethyl paraoxybenzoate, butyl paraoxybenzoate, propyl paraoxybenzoate, methyl paraoxybenzoate, propylene glycol, and phosphoric acid. Examples of antioxidants include citric acid, citric acid derivatives, vitamin C and its derivatives, lycopene, vitamin A, carotenoids, vitamin B and its derivatives, flavonoids, polyphenols, glutathione, selenium, sodium thiosulfate, vitamin E and its derivatives, α-lipoic acid and its derivatives, pycnogenol, flavangenol, superoxide dismutase (SOD), glutathione peroxidase, glutathione-S-transferase, glutathione reductase, catalase, ascorbate peroxidase, and mixtures thereof.

Further, the first liquid medium and the second liquid medium may contain one or more growth factors. Growth factors include, for example, FGF2 and TGF-β1.

Meanwhile, in the method for producing pluripotent stem cells according to the present invention, it is important to culture pluripotent stem cells in a single-cell state in a liquid medium (first liquid medium) that does not substantially contain a PKCβ inhibitor before the culture in a second liquid medium. The first liquid medium used in the method for producing pluripotent stem cells according to the present invention may or may not contain a WNT inhibitor, but preferably does not contain a WNT inhibitor.

In a case in which the first liquid medium contains a WNT inhibitor, the concentration range of the WNT inhibitor is not necessarily limited but may be the same as in the case of the second liquid medium described above.

### 1-4. Effects

According to the method for producing pluripotent stem cells of the present invention, pluripotent stem cells in a single-cell state are cultured in a first liquid medium that does not substantially contain a PKCβ inhibitor, and after the pluripotent stem cells form a cell mass, the suspension culture of the pluripotent stem cells is carried out in a second liquid medium containing a PKCβ inhibitor and a WNT inhibitor, such that a cell mass of pluripotent stem cells that maintains an undifferentiated state of pluripotent stem cells can be formed while preventing cell death of pluripotent stem cells. Therefore, according to the method for producing pluripotent stem cells according to the present invention, it is possible to efficiently produce large quantities of high-quality pluripotent stem cells.

### 2. More Detailed Explanation of Culture Steps

### 2-1. Outline

The step of performing suspension culture using a first liquid medium, the subsequent step of performing suspension culture using a second liquid medium, and other optional steps included in the method for producing pluripotent stem cells according to the present invention will be explained below.

### 2-2. Method

The method in this aspect comprises: a suspension culture step using a first liquid medium and a suspension culture step using a second liquid medium as essential steps; and a maintenance culture step and a collection step as optional steps. When simple expressions such as "suspension culture" and "suspension culture step" are used in the description below, they refer to a culture between the start of a suspension culture step using a first liquid medium and the end of a suspension culture step using a second liquid medium.

### 2-2-1. Maintenance Culture Step

The "maintenance culture step" is a step of culturing a cell population before the suspension culture step or a cell mass obtained after the suspension culture step or the subsequent collection step to proliferate cells while they remain undifferentiated. For the maintenance culture, an animal cell culture method known in the art can be used. For example, it may be the adherent culture, in which cells are cultured while adhering to a culture substrate such as a vessel, carriers, or the like, or the suspension culture.

Hereinafter, although not limited to, an animal cell culture method used in this step and the suspension culture step described later will be exemplified and explained.

### (Cells)

Cells used in this step are cells capable of cell aggregation in the suspension culture. As described in the section "Culture and Medium" in "1-2. Definitions of Terms" above, animal cells are preferable, and human cells are more preferable. In addition, the cell type is pluripotent stem cells, and pluripotent stem cells, like iPS cells and ES cells, are particularly preferable.

### (Culture Vessel)

A culture vessel used for culture is preferably a vessel in which cells have low adhesion to the inner surface of the vessel. An example of a vessel with low cell adhesion to the inner surface of the vessel is a plate subjected to hydrophilic surface treatment with a biocompatible material. For example, Nunclon (trademark) Sphera (Thermo Fisher Scientific Inc.) can be used as a culture vessel.

Examples of the shape of the culture vessel include, but are not particularly limited to, dish-shaped, flask-shaped, well-shaped, bag-shaped, and spinner flask-shaped culture vessels.

The capacity of the culture vessel used can be appropriately selected and is not particularly limited. However, the lower limit of the area of the bottom of the part accommodating the culture medium in the planar view is preferably 0.32 cm² or more, 0.65 cm² or more, 1.9 cm² or more, 3.0 cm² or more, 3.5 cm² or more, 9.0 cm² or more, or 9.6 cm²or more, and the upper limit thereof is preferably 1000 cm² or less, 500 cm² or less, 300 cm² or less, 150 cm² or less, 75 cm² or less, 55 cm² or less, 25 cm² or less, 21 cm² or less, 10 cm² or less, or 3.5 cm² or less.

The capacity of the culture vessel used can be appropriately selected and is not particularly limited. However, the lower limit of the volume capable of accommodating a medium and culturing is preferably 1 mL, 2 mL, 4 mL, 10 mL, 20 mL, 30 mL, 50 mL, 100 mL, 200 mL, 500 mL, 1 L, 3 L, 5 L, 10 L, or 20 L, and the upper limit thereof is preferably 100 L, 50 L, 20 L, 10 L, 5 L, 3 L, 1 L, 500 mL, 200 mL, 100 mL, 50 mL, or 30 mL.

### (Medium)

The amounts of the culture medium and solution may be adjusted as appropriate depending on the culture vessel used. Although not particularly limited, these will be exemplified. For example, in the case of using a 12-well plate (the area of the bottom of the well in the planar view is 3.5 cm² per well), the amount per well can be 0.5 mL or more, 1.5 mL or less, and preferably about 1.3 mL. In the case of using a 6-well plate (the area of the bottom of the well in the planar view is 9.6 cm² per well), the lower limit of the amount per well can be 1.5 mL or more, 2 mL or more, or 3 mL or more, and the upper limit thereof can be 6 mL or less, 5 mL or less, or 4 mL or less. Further, in the case of using a 125-mL Erlenmeyer flask (Erlenmeyer flask with a capacity of 125 mL), the lower limit of the amount per flask can be 10 mL or more, 15 mL or more, 20 mL or more, 25 mL or more, or 30 mL or more, and the upper limit thereof can be 50 mL or less, 45 mL or less, or 40 mL. In the case of using a 500-mL Erlenmeyer flask, the lower limit of the amount per flask can be 100 mL or more, 105 mL or more, 110 mL or more, 115 mL or more, or 120 mL or more, and the upper limit thereof can be 150 mL or less, 145 mL or less, 140 mL or less, 135 mL or less, 130 mL or less, or 125 mL or less. In the case of using a 1000-mL Erlenmeyer flask, the lower limit of the amount per flask can be 250 mL or more, 260 mL or more, 270 mL or more, 280 mL or more, or 290 mL or more, and the upper limit thereof can be 350 mL or less, 340 mL or less, 330 mL or less, 320 mL or less, or 310 mL or less. Furthermore, for example, in the case of using a disposable culture bag with a capacity of 2 L, the lower limit of the amount per bag can be 100 mL or more, 200 mL or more, 300 mL or more, 400 mL or more, 500 mL or more, 600 mL or more, 700 mL or more, 800 mL or more, 900 mL or more, or 1000 mL or more, and the upper limit thereof can be 2000 mL or less, 1900 mL or less, 1800 mL or less, 1700 mL or less, 1600 mL or less, 1500 mL or less, 1400 mL or less, 1300 mL or less, 1200 mL or less, or 1100 mL or less. In the case of using a disposable culture bag with a capacity of 10 L, the lower limit of the amount per bag can be 500 mL or more, 1 L or more, 2 L or more, 3 L or more, 4 L or more, or 5 L or more, and the upper limit thereof can be 10 L or less, 9 L or less, 8 L or less, 7 L or less, or 6 L or less. In the case of using a single-use reactor with a capacity of 1 L, the lower limit of the amount per reactor can be 300 mL or more, 350 mL or more, 400 mL or more, 450 mL or more, or 500 mL or more, and the upper limit thereof can be 1 L or less, 900 mL or less, 800 mL or less, 700 mL or less, or 600 mL or less. In the case of using a stirring blade-type reactor of arbitrary capacity, the capacity can be within the range of the working volume specified by each manufacturer.

### (Seeding Density)

Upon the suspension culture, the cell density (seeding density) of cells seeded in a new medium can be adjusted accordingly in consideration of the culture time, cell state after culture, and the cell count required after culture. Although not limited, in general, the lower limit thereof can be in a range of, for example, 0.01 × 10⁵cells/ mL or more, 0.1 × 10⁵cells/ mL or more, 1 × 10⁵ cells/mL or more, or 2×10⁵ cells/mL or more, and the upper limit thereof can be in a range of, for example, 20 × 10⁵ cells/mL or less, or 10 × 10⁵ cells/mL or less.

### (Culture Conditions)

Culture conditions such as culture temperature, culture time, CO₂ concentration are not particularly limited. Culture may be carried out within the range of ordinary methods in the art. For example, the lower limit of the culture temperature may be 20°C or more or 35°C or more, and the upper limit thereof may be 45°C or less or 40°C or less; and, the culture temperature is preferably 37°C. In addition, the lower limit of the culture time is in a range of 0.5 hours or more or 6 hours or more, and the upper limit thereof is in a range of 7 days or less, 120 hours or less, 96 hours or less, 72 hours or less, or 48 hours or less. The lower limit of the CO₂ concentration during culture may be 0.5% or more, 1% or more, 2% or more, 3% or more, 4% or more, or 4.5% or more, and the upper limit thereof may be 10% or less, 5.5% or less, and preferably 5%. Note that the CO₂ concentration during culture does not need to be constant and may be changed or vary during the culture. In addition, the culture medium can be exchanged at an appropriate frequency. The frequency of medium exchange varies depending on the cell type being cultured. For example, the medium may be exchanged, for example, once or more in 5 days, once or more in 4 days, once or more in 3 days, once or more in 2 days, once or more in 1 day, or twice or more in 1 day, although not limited. Alternatively, the medium may be exchanged continuously using a perfusion process. For a medium exchange, cells may be recovered in the same manner as in the collection step, and then, a fresh culture medium may be added, and cell masses may be gently dispersed, following which the cells may be cultured again. In the case of exchanging the medium using a perfusion process, the cells and the medium may be separated using a filter to retain the cells in the culture system, and then the medium can be exchanged. The frequency and method of medium exchange are not limited to those described above, and an optimal method may be adopted as appropriate.

Further, the timing of terminating the culture and the timing of the medium exchange can also be determined based on, for example, the lactic acid concentration in the medium. Lactic acid is produced by cells during culture and accumulates in the medium. It is known that lactic acid produced by cells, or lactic acid initially contained in the medium, damages cells, and as a result, inhibits the maintenance of the undifferentiated state of pluripotent stem cells, and causes adverse effects such as reducing cell growth, especially cell growth ability after passage. In view of the above, by determining the timing of culture termination and/or the timing and amount of medium exchange based on the lactic acid concentration in the medium, it is possible to avoid the inhibitory effect due to lactic acid on maintaining the undifferentiated state and the effect due to lactic acid on the reduction of cell growth ability.

### (Culture Method)

The fluid state of the medium during culture is arbitrary. Although static culture or flow culture may be used, flow culture is preferable.

"Static culture" refers to culture in a state where the medium is allowed to stand still in a culture vessel. In the adherent culture, this stationary culture is usually employed.

The "flow culture" refers to culture under conditions that allow the medium to flow. In the case of flow culture, a method that allows the medium to flow to promote cell aggregation is preferable. Examples of such a culture method include the rotation culture method, the rocking culture method, the stirring culture method, and any combination thereof.

The "rotation culture method" (such as the shaking culture method) refers to a method for performing culture under conditions that allow a medium to flow such that cells gather at one point due to stress (centrifugal force or centripetal force) by rotational flow. Specifically, culture is performed by rotating a culture vessel accommodating a medium containing cells along a substantially horizontal plane such that a closed orbit such as a circle, an ellipse, a flattened circle, a flattened ellipse, or the like is drawn.

The rotation speed is not particularly limited. However, the lower limit thereof can be, for example, 1 rpm or more, 10 rpm or more, 50 rpm or more, 60 rpm or more, 70 rpm or more, 80 rpm or more, 83 rpm or more, 85 rpm or more, or 90 rpm or more. Meanwhile, the upper limit thereof can be, for example, 200 rpm or less, 150 rpm or less, 120 rpm or less, 115 rpm or less, 110 rpm or less, 105 rpm or less, 100 rpm or less, 95 rpm or less, or 90 rpm or less. The amplitude of a shaker used for rotation culture is not particularly limited. However, the lower limit thereof can be, for example, 1 mm or more, 10 mm or more, or 20 mm or more. Meanwhile, the upper limit thereof can be, for example, 200 mm or less, 100 mm or less, 50 mm or less, or 30 mm or less. The radius of rotation during the rotation culture is not particularly limited; however, the amplitude is preferably set within the above-described range. The lower limit of the radius of rotation can be, for example, 5 mm or more or 10 mm or more, and the upper limit of the radius of rotation can be, for example, 100 mm or less or 50 mm or less. In particular, in the method for producing a cell mass described later or the like, it is preferable to set the rotation condition within the above-described range such that a cell mass having an appropriate size can be easily produced.

The "rocking culture method" refers to a method for performing culture under conditions that impart a rocking flow to a medium by linear reciprocating motion, such as rocking stirring. Specifically, culture is performed by rocking a culture vessel accommodating a medium containing cells within a plane vertical to a substantially horizontal plane. The rocking rate is not particularly limited. For example, given that one round trip is regarded as one time, the lower limit thereof may be two or more times, four or more times, six or more times, eight or more times, or ten or more times of rocking per minute. Meanwhile, the upper limit thereof may be 50 times or less, 25 times or less, 20 times or less, or 15 times or less of rocking per minute. During rocking, it is preferable to give the culture vessel a slight angle, namely a rocking angle, with respect to the vertical plane. The rocking angle is not particularly limited. For example, the lower limit thereof can be 0.1° or more, 2° or more, 4° or more, 6° or more, or 8° or more, and the upper limit thereof can be 20° or less, 18° or less, 15° or less, 12° or less, or 10° or less. In the method for producing a cell mass described later or the like, it is preferable to set the rocking condition within the above-described range such that a cell mass having an appropriate size can be easily produced.

Further, culture can also be performed while stirring by a motion combined with the above-described rotation and rocking motions.

The "stirring culture method" refers to a method of performing culture in which a culture medium in a vessel is stirred using stirring means such as a stirrer bar or stirring blade while the culture vessel is allowed to stand still. For example, stirring culture can be achieved using a spinner flask-like culture vessel equipped with a stirring blade. Such culture vessels are commercially available and can also be used. As long as a commercially available spinner flask-like culture vessel is employed, the amount of cell culture composition recommended by the manufacturer can be suitably used. The stirring speed by the stirring means described above is not particularly limited. However, the lower limit thereof can be 1 rpm or more, 10 rpm or more, 30 rpm or more, 50 rpm or more, or 70 rpm or more, 90 rpm or more, 110 rpm or more, or 130 rpm or more. Meanwhile, the upper limit thereof can be 200 rpm or less or 150 rpm or less.

In the "stirring culture method," it is preferable to control the shear stress applied to cells during culture. Animal cells, including pluripotent stem cells, are generally more susceptible to physical stress than other cells. Therefore, when excessive shear stress is applied to cells during stirring culture, cells might be physically damaged, their growth ability is reduced, and in the case of pluripotent stem cells, they cannot maintain their undifferentiation property.

Physical damage applied to cells during stirring culture may be affected by local shear stress around a stirring blade. Local shear stress around a stirring blade is not limited but is related to, for example, the blade tip speed (circumferential speed at the part where the blade diameter is the largest). The blade tip speed can be obtained as the following formula: Blade diameter [m] × Circumference ratio × Rotational speed [rps] = Blade tip speed [m/s].

Specifically, in suspension culture using a second liquid medium, even when the blade tip speed is extremely high, such as 0.23 m/s or more, stirring culture can be performed while the undifferentiated nature of pluripotent stem cells is maintained.

The blade tip speed is not necessarily limited. However, it is preferably 0.05 m/s or more, preferably 0.08 m/s or more, preferably 0.10 m/s or more, preferably 0.13 m/s or more, preferably 0.17 m/s or more, preferably 0.20 m/s or more, preferably 0.23 m/s or more, preferably 0.25 m/s or more, or preferably 0.30 m/s or more. By setting the blade tip speed within this range, excessive aggregation of cells can be suppressed while maintaining pluripotent stem cells undifferentiated.

The blade tip speed is not necessarily limited. However, it is preferably 1.37 m/s or less, preferably 1.00 m/s or less, preferably 0.84 m/s or less, preferably 0.50 m/s or less, preferably 0.42 m/s or less, preferably 0.34 m/s or less, or preferably 0.30 m/s or less. By setting the blade tip speed within this range, the fluid state of the medium in the culture system can be stabilized while maintaining pluripotent stem cells undifferentiated.

The preferable range of the blade tip speed described above can also be applied to the single-use reactor manufactured by ABLE, which is provided for the suspension culture of pluripotent stem cells. In addition, in stirring culture, when changing the culture scale, the rotational speed of the stirring blade may be determined using a formula of constant Pv, although this is not particularly limited. Pv refers to the power required for stirring per unit volume, and by making Pv the same, stirring culture can be performed similarly on different scales. The formula of constant Pv can be expressed as follows: Rotational speed per unit time [rpm or rps] × (Blade diameter [m])^{2/3} = Constant.

The extent to which the number of cells is increased and the state of the cells in this step may be determined as appropriate depending on the type of cells to be cultured, the purpose of cell aggregation, the type of medium, and the culture conditions.

Hereinafter, although not limited, a culture method in which this step is carried out by an adherent culture method will be exemplified and explained. Here, the descriptions common to the above-described "2-2-1. Maintenance Culture Step" will be omitted, and only the characteristic points of the adherent culture method will be described in detail.

In the adherent culture method, cells are allowed to adhere to a substrate such as a culture vessel and microcarriers, or an external matrix and then cultured. Examples of an external matrix that can be used include, but are not particularly limited to, laminin, vitronectin, gelatin, collagen, and E-cadherin chimeric antibody. For example, a peel-off cell culture plate (Sumitomo Bakelite Co., Ltd.) can be used as a culture vessel. The extent to which the number of cells is increased and the state of the cells in this step may be determined as appropriate depending on the type of cells to be cultured, the type of medium, and the culture conditions. For example, the end point of culture can be set when the coverage of adherent cells on the culture substrate reaches 50% or more, 60% or more, 70% or more, 80% or more, or 90% or more.

### (Post-step Treatment)

After this step, the culture solution and cells are separated by a conventional method, and the cells are collected. At this time, it is preferable to collect cells as single cells by detachment or dispersion treatment. The specific method will be described in detail in the section of single-cell processing of the suspension culture step and in the recovery step, which will be described later. The collected cells may be directly or, if necessary, washed with buffer (including PBS buffer), physiological saline, or a medium (preferably a medium used in the next step or a basal medium) and then subjected to the next step.

### 2-2-2. Suspension Culture Step

As described above, "suspension culture step" includes a suspension culture step using a first liquid medium and a suspension culture step using a second liquid medium. In this section, the suspension culture step using a first liquid medium and the suspension culture step using a second liquid medium will be described; however, the steps basically follow the culture method described in "2-2-1. Maintenance Culture Step" above. Therefore, the descriptions common to the above-described method in the maintenance culture step will be omitted herein, and only the characteristic points of this step will be described in detail.

### (Cells)

Although the cells used in this step are not limited, cells prepared after the maintenance culture step are preferable. As described in the maintenance culture step, the cell type is pluripotent stem cells, and pluripotent stem cells like iPS cells and ES cells are particularly preferable. In addition, the state of the cells when seeded in a first liquid medium is preferably a single-cell state. Being in a single-cell state does not necessarily mean that all of the cell population is in a single-cell state, but rather there may be several cells which have adhered to each other in addition to cells in a single state in the cell population.

The pluripotent stem cells used in this step may be single cell or a cell population (pluripotent stem cell population) consisting of a plurality of cells. In a case in which the pluripotent stem cell is a pluripotent stem cell population, the proportion (percentage) of cells expressing pluripotent stem cell markers (e.g., OCT4, SOX2, NANOG) and/or cells positive for pluripotent stem cell markers in the population is, for example, 90% or more, 91% or more, 92% or more, 93% or more, 94% or more, 95% or more, 96% or more, 97% or more, 98% or more, 99% or more, or 100%.

### (First Liquid Medium)

The first liquid medium is a liquid medium that differs from the second liquid medium described below in that it does not substantially contain a PKCβ inhibitor. As the first liquid medium, any liquid medium capable of performing suspension culture of pluripotent stem cells can be used among the media described in the section of the maintenance culture step described above without restriction.

As used herein, the condition that the first liquid medium does not substantially contain a PKCβ inhibitor is not limited to a case in which the medium does not contain any PKCβ inhibitor or the PKCβ inhibitor in the medium is at or below the detection limit. It should also be understood to include a case in which the PKCβ inhibitor is, for example, at a final concentration of less than 25 nM. In other words, the first liquid medium can have a final concentration of the PKCβ inhibitor of less than 25 nM, less than 23 nM, less than 20 nM, less than 15 nM, less than 10 nM, less than 5 nM, or less than 3 nM. Most preferably, the first liquid medium contains a PKCβ inhibitor at or below the detection limit. As long as the PKCβ inhibitor in the first liquid medium is at or below the detection limit or within the above-described concentration range, the PKCβ inhibitor does not act on cells and the effect of preventing cell death can be exhibited.

For example, in a case in which in the step before the suspension culture of the pluripotent stem cells in the single-cell state in the first liquid medium, culturing the pluripotent stem cells using a medium containing a PKCβ inhibitor (e.g., a second liquid medium) and then if necessary, the pluripotent stem cells in single-cell state are subjected to the production method of the present invention, since the cells are cultured in a medium containing a PKCβ inhibitor (e.g., a second liquid medium) in the previous step, there is a possibility that the PKCβ inhibitor will be brought in when the pluripotent stem cells are seeded into the first liquid medium. However, even when a PKCβ inhibitor is brought in when seeding pluripotent stem cells, this case is one aspect in which the pluripotent stem cells are cultured by the suspension culture in the first liquid medium that does not substantially contain the PKCβ inhibitor, as long as the concentration is within the above-described range.

In addition, depending on the type of pluripotent stem cells cultured in the first liquid medium, culture conditions, and the like, not only the above-described PKCβ inhibitors but also substances having an inhibitory effect on PKCβ may be produced in the medium. Even this case is included in the aspect in which the pluripotent stem cells can be cultured by the suspension culture in the first liquid medium that does not substantially contain the PKCβ inhibitor, as long as the concentration is within the above-described range.

The first liquid medium may have a composition containing a WNT inhibitor but preferably does not contain a WNT inhibitor. At such time, the condition that the first liquid medium does not contain a WNT inhibitor means the condition that the first medium does not substantially contain a WNT inhibitor. As with the PKCβ inhibitor, it is not limited to a case in which the WNT inhibitor in the medium is at or below the detection limit. It should also be understood to include a case in which the WNT inhibitor is, for example, at a final concentration of less than 90 nM. In other words, the first liquid medium can have a final concentration of the WNT inhibitor of less than 90 nM, less than 85 nM, less than 80 nM, less than 75 nM, less than 70 nM, less than 65 nM, less than 60 nM, less than 55 nM, less than 50 nM, less than 45 nM, less than 40 nM, less than 35 nM, less than 30 nM, less than 25 nM, less than 20 nM, less than 15 nM, less than 10 nM, less than 5 nM or less than 3 nM. Most preferably, the first liquid medium contains a WNT inhibitor at or below the detection limit. As long as the WNT inhibitor in the first liquid medium is at or below the detection limit or within the above-described concentration range, the WNT inhibitor does not act on cells and the effect of further preventing cell death can be exhibited.

Also, regarding the WNT inhibitor, even when the WNT inhibitor is brought into the medium when seeding pluripotent stem cells or even when substances that inhibit WNT signals are produced in the medium depending on the type and/or culture conditions of pluripotent stem cells, this case is an aspect in which the suspension culture of pluripotent stem cells is performed in the first liquid medium that does not contain a WNT inhibitor as long as the concentration range of the WNT inhibitor is within the above-described range.

### (Second Liquid Medium)

The second liquid medium is characterized by containing a PKCβ inhibitor and a WNT inhibitor. The type of medium is not limited as long as the medium contains a PKCβ inhibitor and a WNT inhibitor and can proliferate and/or maintain cells.

The concentrations of the PKCβ inhibitor and WNT inhibitor contained in the second liquid medium are set as described in "1-3. Configuration" above. There is no particular restriction on the method for adding a PKCβ inhibitor and a WNT inhibitor, as long as the concentrations of the PKCβ inhibitor and the WNT inhibitor at the start of culture using a second liquid medium are within the ranges described above. For example, the second liquid medium may be prepared by directly adding one or more types of PKCβ inhibitors and WNT inhibitors to a medium such that the total amount thereof falls within the above-described concentration range.

### (Single-cell Processing)

As used herein, "single-cell processing (in a single-cell state)" refers to the dispersion of cell aggregates in which a plurality of cells adhere to or aggregate with each other, such as monolayer cell fragments or cell masses, into a single, free cell state. In the suspension culture of the pluripotent stem cells using the first liquid medium, the cell aggregates are dispersed, and thus, pluripotent stem cells in a single free state are cultured by the suspension culture.

A detachment agent and/or a chelating agent is used for single-cell processing. Examples of a detachment agent that can be used include, but are not limited to, trypsin, collagenase, pronase, hyaluronidase, elastase, and, in addition, commercially available products such as Accutase (registered trademark), Accumax (registered trademark), TrypLE (trademark) Express Enzyme (Life Technologies Japan Ltd.), TrypLE (trademark) Select Enzyme (Life Technologies Japan Ltd.), and Dispase (registered trademark). In the case of using trypsin for single-cell processing, the lower limit of the concentration in the solution is not particularly limited as long as the cell population can be dispersed. The lower limit of the concentration may be, for example, 0.15% by volume or more, 0.18% by volume or more, 0.20% by volume or more, or 0.24% by volume or more. Meanwhile, the upper limit of the concentration in the solution is not particularly limited as long as, for example, the concentration does not cause cells per se to be dissolved. The upper limit of the concentration may be, for example, 0.30% by volume or less, 0.28% by volume or less, or 0.25% by volume or less. The treatment time depends on the concentration of trypsin. However, the lower limit thereof is not particularly limited as long as the cell population is well dispersed by the action of trypsin within the time. The lower limit of the treatment time may be, for example, 5 minutes or more, 8 minutes or more, 10 minutes or more, 12 minutes or more, or 15 minutes or more. Meanwhile, the upper limit of treatment time is not particularly limited as long as, for example, the time does not cause cells per se to be dissolved by the action of trypsin. The upper limit of the treatment time may be, for example, 30 minutes or less, 28 minutes or less, 25 minutes or less, 22 minutes or less, 20 minutes, or 18 minutes or less. In the case of using a commercially available detachment agent, it may be used at a concentration that allows the cells to be dispersed into a single state, as described in the attached protocol. After treatment with the detachment agent and/or chelating agent, single-cell processing can be promoted by lightly physically treating the cells. This physical treatment is not limited but includes, for example, a method for pipetting cells together with the solution a plurality of times. Further, the cells may be passed through a strainer or mesh, if necessary.

The obtained cells in a single-cell state can be collected by removing the supernatant containing the detachment agent via standing or centrifugation. The collected cells may be washed, if necessary. The conditions for centrifugation and the washing method may be the same as described above.

### (Culture Method)

According to the method for producing pluripotent stem cells of the present invention, at first, pluripotent stem cells in a single-cell state are cultured by suspension culture in the first liquid medium, and after the pluripotent stem cells form a cell mass, suspension culture of the pluripotent stem cells is performed in the second liquid medium. Here, in both the suspension culture using the first liquid medium and the suspension culture using the second liquid medium, the culture method is preferably flow culture in which the medium is flown.

In the suspension culture step using the first liquid medium, conditions such as the culture vessel, medium amount, culture temperature, seeding density, rotation conditions, and stirring conditions described in the section of the maintenance culture step can be applied. The suspension culture using the first liquid medium is performed until the pluripotent stem cells during culture form a cell mass.

In this method, whether or not pluripotent stem cells have formed a cell mass (whether they are in a single state or a cell mass) can be judged by sampling the culture medium during culture and observing cells contained in the culture solution using a microscope or other means. Specifically, among aggregates in which cells aggregate, the cell mass can be defined as a cell mass that has the lower limit of the maximum width of the aggregate of 35 µm or more, 40 µm or more, 45 µm or more, 50 µm or more, 55 µm or more, or 60 µm or more. In a case in which the sampled medium contains a cell mass defined as falling within the range, it can be considered the final stage of the suspension culture step using the first liquid medium. Alternatively, when among the observed cell aggregates, a cell mass defined as falling within the above-described range accounts for 10% or more, 20% or more, 30% or more, 40% or more, 50% or more, 60% or more, 70% or more, 75% or more, 80% or more, 85% or more, 90% or more, 95% or more, 98% or more, or 100% or more on a weight basis compared to the cell amount at the start of culture, it can be considered the final stage of the suspension culture step using the first liquid medium.

In this method, whether or not pluripotent stem cells have formed a cell mass can be judged based on whether or not a cell aggregate that meets a predetermined size is observed or whether or not a cell aggregate that meets a predetermined size exists at a level exceeding a predetermined proportion. More specifically, it can be judged based on a criterion that the suspension culture using the first liquid medium can be terminated when a cell mass having a maximum width of 50 µm or more accounts for 60% or more on a weight basis.

Alternatively, whether or not a cell mass has been formed in the suspension culture of pluripotent stem cells using the first liquid medium can also be determined based on the elapsed time from the start of the culture. This is a judgment based on the findings that the growth rate and cell mass formation rate of pluripotent stem cells are the same to some extent, depending on the type of pluripotent stem cells to be cultured, medium components, culture conditions, and the like. In general, the cell mass defined above is formed 8 hours, 8.5 hours, 9 hours, 9.5 hours, 10 hours, 10.5 hours, 11 hours, 11.5 hours, 12 hours, 13 hours, 14 hours, 15 hours, 16 hours, 17 hours, 18 hours, 19 hours, 20 hours, 21 hours, 22 hours, 23 hours, 24 hours, or 48 hours after seeding the cells in a single-cell state. Therefore, the suspension culture using the first liquid medium can be considered the final stage 8 hours, 8.5 hours, 9 hours, 9.5 hours, 10 hours, 10.5 hours, 11 hours, 11.5 hours, 12 hours, 13 hours, 14 hours, 15 hours, 16 hours, 17 hours, 18 hours, 19 hours, 20 hours, 21 hours, 22 hours, 23 hours, 24 hours, or 48 hours after starting the suspension culture of pluripotent stem cells in the first liquid medium, given that a cell mass has been formed. In many cases, the suspension culture using the first liquid medium can be terminated when 24 hours have passed since the start of suspension culture of pluripotent stem cells in the first liquid medium.

As described above, after a cell mass has been formed in the suspension culture step using the first liquid medium, the step proceeds to the suspension culture step using the second liquid medium. In particular, according to the method for producing pluripotent stem cells according to the present invention, the undifferentiated state of pluripotent stem cells can be maintained by performing the suspension culture of pluripotent stem cells using the second liquid medium containing a PKCβ inhibitor and a WNT inhibitor.

The suspension culture step using the second liquid medium may be initiated by adding a PKCβ inhibitor and optionally a WNT inhibitor to the medium or by seeding the cell masses collected from the first liquid medium into the second liquid medium, after the end of the suspension culture step using the first liquid medium. In addition, the PKCβ inhibitor or WNT inhibitor may be added such that the concentration thereof in the culture solution gradually increases.

At such time, it is possible to take out a part of the pluripotent stem cells during culture and to confirm whether the undifferentiated state is maintained. For example, it is possible to confirm whether the undifferentiated state is maintained by measuring the expression level of pluripotent stem cell markers expressed in pluripotent stem cells taken out during culture. Examples of pluripotent stem cell markers can include Alkaline Phosphatase, NANOG, OCT4, SOX2, TRA-1-60, c-Myc, KLF4, LIN28, SSEA-4, and SSEA-1, as described above. Examples of the method for detecting these pluripotent stem cell markers include flow cytometry, as described above.

When the positive rate of pluripotent stem cell markers among pluripotent stem cells taken out during culture is preferably 80% or more, more preferably 90% or more, still more preferably 91% or more, even more preferably 92% or more, yet more preferably 93% or more, yet even more preferably 94% or more, yet even more preferably 95% or more, yet even more preferably 96% or more, yet even more preferably 97% or more, yet even more preferably 98% or more, yet even more preferably 99% or more, or yet even more preferably 100%, it can be determined that the cells remain undifferentiated.

In addition, in the suspension culture step using the second liquid medium, it is possible to confirm whether the undifferentiated state is maintained by measuring the expression level of three germ layer markers (endodermal cell marker, mesoderm cell marker, and ectodermal cell marker) in the pluripotent stem cells taken out during culture. In other words, the respective positive rates of these endodermal cell marker, mesodermal cell marker, and ectodermal cell marker are preferably 20% or less, more preferably 10% or less, still more preferably 9% or less, even more preferably 8% or less, yet more preferably 7% or less, yet even more preferably 6% or less, yet even more preferably 5% or less, yet even more preferably 4% or less, yet even more preferably 3% or less, yet even more preferably 2% or less, yet even more preferably 1% or less, or yet even more preferably the detection limit or less, it can be determined that the undifferentiated state is maintained.

"Endodermal cell markers" refer to genes specific to endodermal cells. Examples thereof can include SOX17, FOXA2, CXCR4, AFP, GATA4, and EOMES. Endodermal cells differentiate into tissues of organs such as the digestive tract, lung, thyroid, pancreas and liver, and cells of secretory glands opening to the digestive tract, peritoneum, pleura, larynx, auditory tube, trachea, bronchi, urinary tracts (bladder most of the urethra, part of ureters), and the like.

"Mesodermal cell markers" refer to genes specific to mesodermal cells. Examples thereof can include TBXT (BRACHYURY), MESP1, MESP2, FOXF1, HAND1, EVX1, IRX3, CDX2, TBX6, MIXL1, ISL1, SNAI2, FOXC1, and PDGFRα. Mesodermal cells differentiate into body cavities and mesothelium lining them, muscle, skeleton, skin dermis, connective tissue, heart, blood vessels (including vascular endothelium), blood (including blood cells), lymph vessels, spleen, kidneys, ureters, gonads (testis, uterus, gonadal epithelium), and the like.

"Ectodermal cell markers" refer to genes specific to ectodermal cells. Examples thereof can include FGF5, NESTIN, SOX1, and PAX6. Ectodermal cells form the epidermis of the skin and the epithelium of the terminal urethra in males, hair, nails, skin glands (including mammary and sweat glands), sensory organs (including the terminal epithelium of the oral cavity, pharynx, nose, and rectum, salivary glands), lens, peripheral nervous system, and the like. In addition, part of the ectoderm is invaginated into grooves during development to form the neural tube and also serves as the origin of neurons and melanocytes in the central nervous system, such as the brain and spinal cord.

The expression levels of these three germ layer markers (endodermal cell marker, mesoderm cell marker, and ectoderm cell marker) can be measured by any detection method in the art. Examples of the method for measuring the expression of three germ layer markers (endodermal cell marker, mesodermal cell marker, and ectodermal cell marker) include, but are not limited to, quantitative real-time PCR analysis, the RNA-Seq method, and hybridization methods using northern hybridization or DNA array. In the quantitative real-time PCR analysis, the expression level of the marker gene to be measured is converted into the relative expression level with respect to the expression level of an internal standard gene, and the expression level of the marker gene can be evaluated based on the relative expression level. Examples of an internal standard gene include the glyceraldehyde-3-phosphate dehydrogenase (GAPDH) gene and the β-actin (ACTB) gene.

Meanwhile, in the case of performing the suspension culture of pluripotent stem cells in the second liquid medium, the presence of a PKCβ inhibitor and a WNT inhibitor can reduce the adverse effects of lactic acid as described in the section of (Culture Conditions) of "2-2-1. Maintenance Culture Step." In other words, it is possible to maintain the undifferentiated state and cell growth ability even with a higher lactic acid concentration than in the typical cell culture. Specifically, even when the lactic acid concentration in the culture solution reaches 5 mM or more, culture can be carried out appropriately. Further, even when the lactic acid concentration in the culture solution reaches 7 mM or more, 9 mM or more, 10 mM or more, 11 mM or more, or 12 mM or more, culture can be performed appropriately.

At the same time, it is preferable that the lactic acid concentration accumulated in the culture solution is 15 mM or less. In particular, the lactic acid concentration accumulated in the culture solution is preferably 14 mM or less, 13 mM or less, 12 mM or less, or 10 mM or less. As long as the lactic acid concentration accumulated in the culture solution is within this range, adverse effects on cells can be avoided.

As described above, the timing of culture termination or the timing of medium exchange in the step of performing the suspension culture of pluripotent stem cells in the second liquid medium can be set to until the lactic acid concentration accumulated in the medium reaches, for example, 15 mM, 14 mM or less, 13 mM or less, 12 mM or less, or 10mM or less. In another embodiment, the timing of culture termination or the timing of medium exchange can be set at a time point when the lactic acid concentration accumulated in the medium is in a low concentration range. In addition, in the case of medium exchange by the perfusion process, the perfusion rate can be adjusted such that the lactic acid concentration accumulated in the medium can be maintained at, for example, 14 mM or less, 13 mM or less, 12 mM or less, 11 mM or less, 10 mM or less, 9 mM or less, 8 mM or less, or 7 mM or less.

Alternatively, in the step of performing the suspension culture of pluripotent stem cells in the second liquid medium, the timing of culture termination or the timing or amount of medium exchange can also be determined based on the size of the cell mass. The upper limit of the size of the cell mass can be, for example, 800 µm, 700 µm, 600 µm, 500 µm, 400 µm, 300 µm, 250 µm, or 200 µm. The lower limit of the size of the cell mass can be, for example, 50 µm, 100 µm, or 150 µm. A cell mass (preferably a cell mass having a smaller size) within this range is preferable as a growth environment for cells because oxygen and nutrients are easily supplied to the cells inside. In a case in which the medium sampled in the suspension culture using the second liquid medium contains a cell mass of such a size, it can be considered the final stage of the suspension culture step using the second liquid medium. Alternatively, when among the observed cell masses, the cell mass defined as falling within the above-described range accounts for 10% or more, 20% or more, 30% or more, 40% or more, 50% or more, 60% or more, 70% or more, 75% or more, 80% or more, 85% or more, 90% or more, 95% or more, 98% or more, or 100% on a volume basis, it can be considered the final stage of the suspension culture step using the second liquid medium.

Alternatively, the suspension culture of pluripotent stem cells using the second liquid medium can also be determined based on the elapsed time from the start of the culture, as with the suspension culture using the first liquid medium. This is also a judgment based on the findings that the growth rate and cell mass formation rate of pluripotent stem cells are the same to some extent, depending on the type of pluripotent stem cells to be cultured, medium components, culture conditions, and the like. The suspension culture using the second liquid medium can be considered the final stage 48 hours, 60 hours, 72 hours, 84 hours, 96 hours, 108 hours, 120 hours, 132 hours, or 144 hours after starting the suspension culture of pluripotent stem cells in the second liquid medium, given that a cell mass of the above-described size range has been formed.

In the suspension culture using the second liquid medium, it is possible to reduce the adverse effects (decreased growth ability, difficulty in maintaining an undifferentiated state) due to physical damage applied to the cell mass of pluripotent stem cells. In other words, in a case in which pluripotent stem cells are cultured by the suspension culture in the presence of a PKCβ inhibitor and a WNT inhibitor, their undifferentiated state can be maintained or the cell growth ability can be maintained, even under conditions where greater physical damage is applied than in the typical cell culture. It means that in the suspension culture using the second liquid medium, culture is carried out at the same or higher blade tip speed, stirring speed, required stirring power per unit volume (Pv), or the like, compared to the suspension culture using the first liquid medium.

Specifically, in the suspension culture using the second liquid medium, even when the blade tip speed is extremely high, such as 0.23 m/s or more, stirring culture can be performed while the undifferentiated nature of pluripotent stem cells is maintained.

As described above, a population of cell masses produced through the suspension culture step using the first liquid medium and the suspension culture step using the second liquid medium is characterized in that the proportion of living cells (survival rate) among the cells constituting the population is high. In other words, a population of cell masses produced through the suspension culture using the first liquid medium and the suspension culture using the second liquid medium can exhibit a superior survival rate compared to a population of cell masses produced only by the suspension culture using the second liquid medium. For example, the survival rate of a population of cell masses produced through the suspension culture using the first liquid medium and the suspension culture using the second liquid medium can be 50% or more, 60% or more, 70% or more, 80% or more, or 90% or more.

After the suspension culture using the second liquid medium, the obtained cell mass can be used as it is depending on the purpose, or the obtained cell mass is made into single cells by the above-described "single-cell processing" and then the obtained single cells or group of cells may be applied to the method for producing pluripotent stem cells according to the present invention again. In other words, it is also possible to perform the suspension culture of the obtained pluripotent stem cells in a single-cell state using the first liquid medium again, and then, when cell masses are formed, perform the suspension culture using the second liquid medium again.

### 2-2-3. Collection Step

The "collection step" is a selection step in the method of the present invention, which is a step of collecting the cultured cells or cell masses from a culture solution after the maintenance culture step, the suspension culture step using the first liquid medium, or the suspension culture step using the second liquid medium.

As used herein, "collection (of cells)" refers to obtaining cells by separating the culture solution and the cells. A method for collecting cells may follow a conventional method used in cell culture methods in the art, which is not particularly limited.

Cells are present in the culture solution in a suspended state after the suspension culture step using the first liquid medium and the suspension culture step using the second liquid medium. Therefore, the collection of cells can be achieved by removing the liquid component of the supernatant via standing or centrifugation. Cells can also be collected using a filtration filter, a hollow fiber separation membrane, or the like. In the case of removing the liquid component via standing, the vessel containing the culture solution is allowed to stand still for about 5 minutes, and the supernatant may be removed while leaving the sedimented cells or cell masses. For centrifugation, the centrifugal acceleration and the treatment time may be such that the centrifugal force does not damage the cells. For example, the lower limit of the centrifugal acceleration is not particularly limited as long as cells can be sedimented. The lower limit thereof may be, for example, 100 × g or more, 300 × g or more, 800 × g or more, or 1000 × g or more. Meanwhile, the upper limit thereof may be any speed at which the centrifugal force does not or hardly damage the cells. The upper limit thereof may be, for example, 1600 × g or less, 1500 × g or less, or 1400 × g or less. The lower limit of the treatment time is not particularly limited as long as it is the time during which the above-described centrifugal acceleration can sediment cells. The lower limit thereof may be, for example, 30 seconds or more, 1 minute or more, 3 minutes or more, or 5 minutes or more. The upper limit thereof may be any time during which the above-described centrifugal acceleration does not or hardly damage the cells. The upper limit thereof may be, for example, 10 minutes or less, 8 minutes or less, 6 minutes or less, or 30 seconds or less. In the case of removing the liquid component by filtration, for example, the culture solution may be passed through a nonwoven fabric or a mesh filter to remove the filtrate, thereby collecting the remaining cell aggregates. In the case of removing the liquid component by a hollow fiber separation membrane, for example, the culture solution and the cells may be separated and collected using an apparatus equipped with a hollow fiber separation membrane, such as a cell concentration and washing system (KANEKA CORPORATION).

The collected cells and cell mass can be washed as necessary. The washing method is not limited. For example, the washing method may be carried out as with the washing method described in "Post-step Treatment" in the above-described maintenance culture step. Buffer (such as PBS buffer), physiological saline, or a medium (preferably basal medium) may be used as a washing liquid.

### Examples

Hereinafter, the method for producing pluripotent stem cells will be described in more detail with reference to Examples. However, the scope of the present invention is not intended to be limited by the following Examples.

### <Reference Example 1: Adherent Culture of Human iPS Cell Line 201B7>

A human iPS cell line 201B7 (Center for iPS Cell Research and Application, Kyoto University (CiRA)) was seeded on a cell culture dish coated with Vitronectin (VTN-N) Recombinant Human Protein, Truncated (Thermo Fisher Scientific Inc.) at 0.5 µg/cm², followed by adherent culture at 37°C in a 5% CO₂ atmosphere. StemFit (registered trademark) AK02N (AJINOMOTO CO., INC.) was used as a medium, and medium exchange was carried out daily. Y-27632 (FUJIFILM Wako Pure Chemical Corporation) was added to the medium, thereby yielding a final concentration of 10 µM only during cell seeding.

### <Reference Example 2: Suspension Culture of Human iPS Cell Line 201B7>

Human iPS cell line 201B7 after adherent culture in the same manner as in Reference Example 1 was treated with Accutase for 5 minutes to be detached from the culture surface, and the cells were dispersed into a single state by pipetting. The cells were suspended in StemFit (registered trademark) AK02N medium containing Y-27632 at a final concentration of 10µM, and some of the cells were stained with trypan blue, followed by measurement of the viable cell count. A cell suspension was prepared using StemFit (registered trademark) AK02N medium containing Y-27632 at a final concentration of 10 µM such that the cell suspension included 2×10⁵ cells per 1 mL. The cell suspension in an amount of 1 mL was seeded per well in a 12-well plate for suspension culture (Sumitomo Bakelite Co., Ltd.). The plate seeded with cells was rotated on a rotary shaker at a speed of 98 rpm along a horizontal plane in a circle with a rotation width (diameter) of 25 mm, during which the suspension culture was performed at 37°C in a 5% CO₂ environment.

The day when the cells were seeded was defined as day 0 of culture, passage was performed on day 5 of culture, and suspension culture was performed until day 10 of culture. Medium exchange was carried out every day. As for the method of medium exchange, the entire amount of the medium containing cell masses was collected into a centrifuge tube, and the tube was allowed to stand for about 5 minutes, thereby settling the cell masses. Then, the culture supernatant was removed, and the residue was gently resuspended in StemFit (registered trademark) AK02N medium, and returned to the original well.

Upon medium exchange, Y-27632 was added to the medium at a final concentration of 5 µM on days 1 and 6 of culture, and Y-27632 was added to the medium at a final concentration of 2 µM on days 2 and 7 of culture. On day 5 of culture, the cell masses and culture supernatant were collected from the wells into a centrifuge tube and allowed to stand for about 5 minutes, thereby settling the cell masses. Then, the culture supernatant was removed.

After adding 1 mL of Accutase to the cell masses, the cell masses were treated at 37°C for 10 minutes and then dispersed into a single-cell state by pipetting. The cells were suspended in StemFit (registered trademark) AK02N medium containing Y-27632 at a final concentration of 10µM, and some of the cells were stained with trypan blue, followed by measurement of the viable cell count. A cell suspension was prepared using StemFit (registered trademark) AK02N medium containing Y-27632 at a final concentration of 10 µM such that the cell suspension included 2×10⁵ cells per 1 mL. Then, cell seeding was performed in the same manner as in the case of day 0 to continue the suspension culture.

### <Reference Example 3: Addition of WNT Inhibitor in Suspension Culture of Human iPS Cell Line 201B7>

Suspension culture was performed in the same manner as in Reference Example 2, except that IWR-1-endo was added as a WNT inhibitor at a final concentration of 20 µM to the seeding medium and the medium exchanged for new medium for suspension culture.

### <Reference Example 4: Addition of PKCβ Inhibitor in Suspension Culture of Human iPS Cell Line 201B7>

Suspension culture was performed in the same manner as in Reference Example 2, except that GF109203X was added as a PKCβ inhibitor at a final concentration of 3 µM to the seeding medium and the medium exchanged for new medium for suspension culture.

### <Reference Example 5: Addition of WNT Inhibitor and PKCβ Inhibitor in Suspension Culture of Human iPS Cell Line 201B7>

Suspension culture was performed in the same manner as in Reference Example 2, except that IWR-1-endo was added as a WNT inhibitor at a final concentration of 20 µM and GF109203X was added as a PKCβ inhibitor at a final concentration of 3 µM to the seeding medium and the medium exchanged for new medium for suspension culture.

### <Evaluation Example 1: Quantitative Real-Time PCR Analysis>

The cells on day 10 of culture in Reference Examples 1, 2, 3, 4, and 5 were dissolved using TRIzol (trademark) Reagent (Thermo Fisher Scientific Inc.). Total RNA was isolated and purified from the solution of the cells dissolved with TRIzol (trademark) Reagent using PureLink (registered trademark) RNA Mini Kit (Thermo Fisher Scientific Inc.). The concentration of purified RNA was measured using BioSpec-nano (SHIMADZU CORPORATION), thereby fractionating 500 ng of purified RNA. The amount of fractionated RNA was adjusted to 10 µL with the addition of 2 µL of ReverTra Ace (registered trademark) qPCR RT Master mix (TOYOBO CO., LTD.) and Rnase Free dHzO. cDNA synthesis was performed using SimpliAmp Thermal Cycler (Thermo Fisher Scientific Inc.). Reaction conditions for cDNA synthesis included carrying out a reaction continuously at 37°C for 15 minutes, at 50°C for 5 minutes and at 98°C for 5 minutes, and then cooling to 4°C. The synthesized cDNA solution was diluted 100-fold with 10 mM Tris-HCl pH 8.0 (NACALAI TESQUE, INC.) and added at 5 µL/well to a 384-well PCR plate (Thermo Fisher Scientific Inc.). KOD SYBR (registered trademark) qPCR Mix (TOYOBO CO., LTD.), a forward primer adjusted to 50 µM, a reverse primer adjusted to 50 µM, and DEPC-treated water (NACALAI TESQUE, INC.) were mixed at a ratio of 100:1:1:48. This liquid mixture was added at 15 µL/well to the 384-well PCR plate, followed by mixing. GAPDH, OCT4, T, SOX17, and PAX6 were used as primers. The 384-well PCR plate was centrifuged, thereby removing air bubbles in the wells. Quantitative real-time PCR analysis was performed using QuantStudio 7 Flex Real-Time PCR System (Thermo Fisher Scientific Inc.). Table 1 lists the reaction conditions.

**[Table 1]**

| Step | Temperature | Time | Number of cycles |
|---|---|---|---|
| 1. Initial denaturation | 98°C | 1 min | - |
| 2. Denaturation | 98°C | 15 sec | 5 cycles |
| 3. Annealing, elongation | 68°C | 30 sec | |
| 4. Denaturation | 98°C | 15 sec | 40 cycles |
| 5. Annealing | 60°C | 10 sec | |
| 6. Elongation | 68°C | 30 sec | |
| 7. Melting curve | 95°C | 15 sec | - |
| | 60°C | 1 min | |
| | 98°C | 15 sec | |

The base sequences of the primers used for quantitative real-time PCR analysis are shown below.
ACTB (Forward): 5'-CCTCATGAAGATCCTCACCGA-3' (SEQ ID NO: 1)
ACTB (Reverse): 5'-TTGCCAATGGTGATGACCTGG-3' (SEQ ID NO: 2)
OCT4 (Forward): 5'-AGTGGGTGGAGGAAGCTGACAAC-3' (SEQ ID NO: 3)
OCT4 (Reverse): 5'-TCGTTGTGCATAGTCGCTGCTTGA-3' (SEQ ID NO: 4)
SOX2 (Forward): 5'-CACCAATCCCATCCACACTCAC-3' (SEQ ID NO: 5)
SOX2 (Reverse): 5'-GCAAAGCTCCTACCGTACCAC-3' (SEQ ID NO: 6)
NANOG (Forward): 5'-AGCCTCCAGCAGATGCAAGAACTC-3' (SEQ ID NO: 7)
NANOG (Reverse): 5'-TTGCTCCACATTGGAAGGTTCCCA-3' (SEQ ID NO: 8)
TBXT (Forward): 5'-TCACAAAGAGATGATGGAGGAAC-3' (SEQ ID NO: 9)
TBXT (Reverse): 5'-ACATGCAGGTGAGTTGTCAG-3' (SEQ ID NO: 10)
SOX17 (Forward): 5'-ATCTGCACTTCGTGTGCAAG-3' (SEQ ID NO: 11)
SOX17 (Reverse): 5'-GAGTCTGAGGATTTCCTTAGCTC-3' (SEQ ID NO: 12)
PAX6 (Forward): 5'-AGGAATGGACTTGAAACAAGG-3' (SEQ ID NO: 13)
PAX6 (Reverse): 5'-GCAAAGCTTGTTGATCATGG-3' (SEQ ID NO: 14)

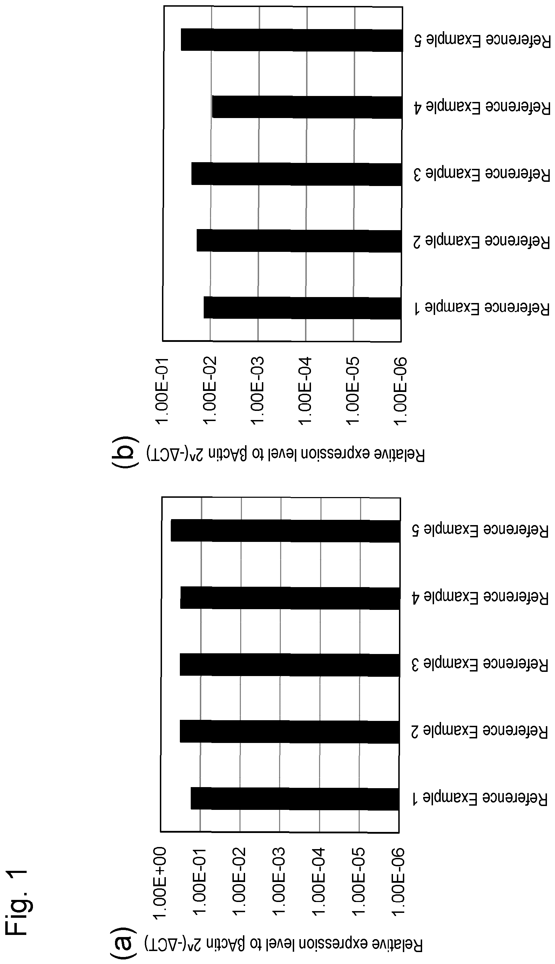

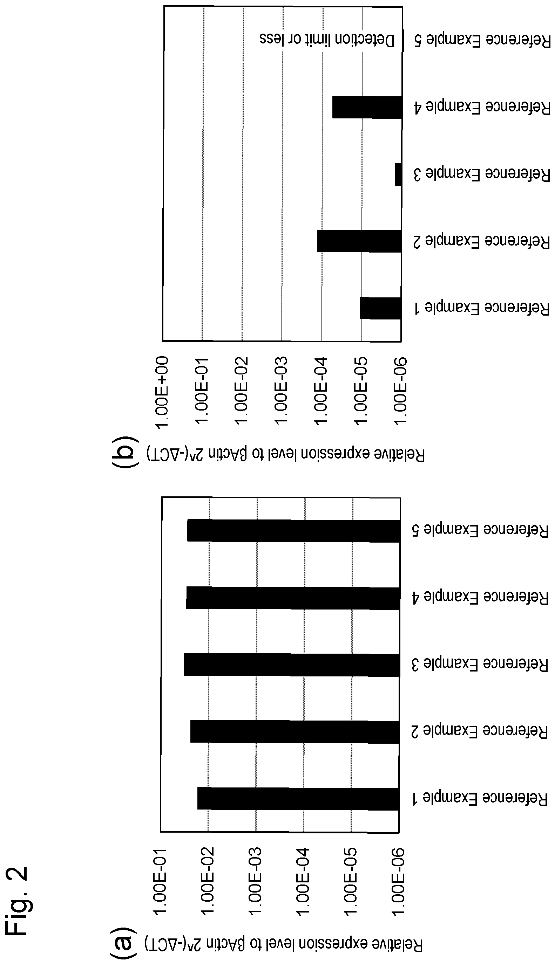

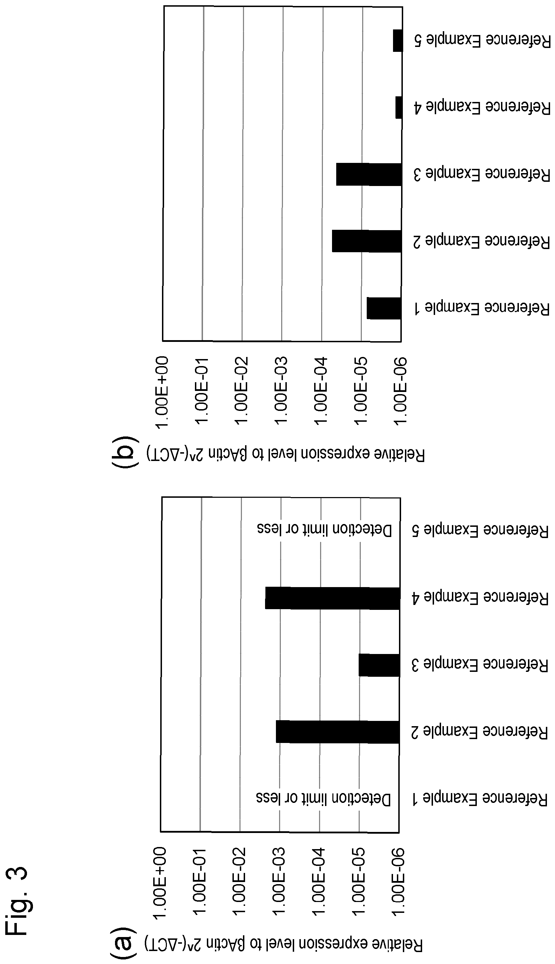

Figures 1 to 3 show the measurement results of gene expressions. Figures 1(a) and 1(b) show the measurement results of the gene expressions of OCT4 and NANOG. Figures 2(a) and 2(b) show the measurement results of the gene expressions of SOX2 and TBXT. Figures 3(a) and 3(b) show the measurement results of the gene expressions of SOX17 and PAX6. In Reference Example 1, in which the adherent culture was performed, the expression levels of TBXT (mesodermal marker gene), SOX17 (endodermal marker gene), and PAX6 (ectodermal marker gene) were low. Thus, the undifferentiated state of the pluripotent stem cells was maintained.

Meanwhile, in Reference Example 2, in which the suspension culture was performed, the expression levels of TBXT, SOX17, and PAX6 increased compared to Reference Example 1. This indicated that in the suspension culture, spontaneous differentiation into mesoderm, endoderm, and ectoderm occurred, and the undifferentiated state of the pluripotent stem cells could not be maintained. In Reference Example 3, in which the suspension culture was performed with the addition of the WNT inhibitor, although the expression levels of TBXT and SOX17 could be suppressed to the expression levels acceptable for the pluripotent stem cells as with Reference Example 1, the expression level of PAX6 was greater than that in Reference Example 1. Thus, there was no improvement compared to Reference Example 2. In Reference Example 4, in which the suspension culture was performed with the addition of the PKCβ inhibitor, although the expression level of PAX6 could be suppressed to an extent comparable to that in Reference Example 1, the expression levels of TBXT and SOX17 were greater than those in Reference Example 1 and thus are high expression levels as with Reference Example 2. In Reference Example 5, in which both the WNT inhibitor and the PKCβ inhibitor were added, the expression levels of TBXT, SOX17, and PAX6 were comparable to those in Reference Example 1.

The above results showed that in the suspension culture, the WNT inhibitor can effectively suppress spontaneous differentiation into mesoderm and endoderm but has a weak suppressive effect on ectoderm differentiation. Further, it was shown that in the suspension culture, the PKCβ inhibitor can suppress spontaneous differentiation into ectoderm but undesirably promotes mesoderm and endoderm differentiation. Meanwhile, it was revealed that when the suspension culture is performed with the addition of both the WNT inhibitor and the PKCβ inhibitor, spontaneous differentiation into all mesoderm, endoderm, and ectoderm can be suppressed.

In other words, it was revealed that to effectively suppress the spontaneous differentiation of the pluripotent stem cells into the mesoderm, endoderm, and ectoderm and maintain the undifferentiated state of the pluripotent stem cells, adding either a WNT inhibitor or a PKCβ inhibitor is not sufficient, and therefore, only by adding both a WNT inhibitor and a PKCβ inhibitor, spontaneous differentiation into all mesoderm, endoderm, and ectoderm can be suppressed and the undifferentiated state of the pluripotent stem cells can be maintained.

<Reference Example 6: Suspension Culture with Addition of WNT Inhibitor and PKCβ Inhibitor>

Suspension culture was performed in the same manner as in Reference Example 2, except that IWR-1-endo was added as a WNT inhibitor at a final concentration of 20 µM, that LY-333531 was added as a PKCβ inhibitor at a final concentration of 1 µM to the seeding medium and that the medium exchanged for new medium for suspension culture, and the cell line used was 1383D6 line.

### <Reference Example 7: Suspension Culture of Human iPS Cell Line 1383D6>

Suspension culture was performed in the same manner as in Reference Example 2, except that the cell line used was 1383D6 line.

### <Evaluation Example 2: Confirmation of Growth Ability>

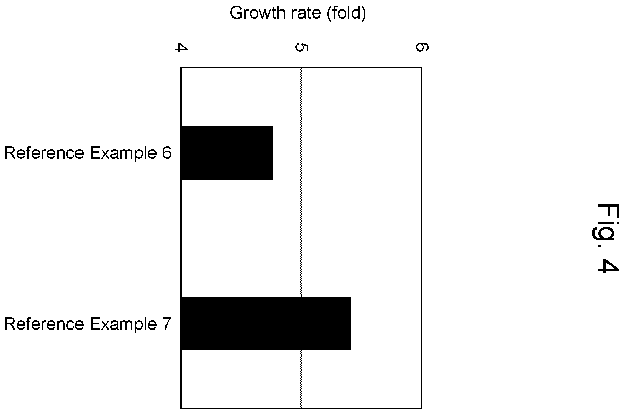

The cells of Reference Examples 6 and 7 on day 4 of culture were processed into the single-cell state in the same manner described in Reference Example 2, and the number of cells was counted. The growth rate relative to the number of seeded cells was calculated. Figure 4 shows the results. As depicted in Figure 4, it was shown in the case of suspension culture with the addition of a WNT inhibitor and a PKCβ inhibitor (Reference Example 6), the growth rate thereof declines compared to the case in which a WNT inhibitor and a PKCβ inhibitor are not added (Reference Example 7). It is possible that the WNT inhibitor and the PKCβ inhibitor caused cell death or growth inhibition.

### <Example 1: Seeding in Suspension Culture in Absence of PKCβ Inhibitor and WNT Inhibitor>

A human iPS cell line 1383D6 (Center for iPS Cell Research and Application, Kyoto University (CiRA)) was seeded on a cell culture dish coated with Vitronectin (VTN-N) Recombinant Human Protein, Truncated (Thermo Fisher Scientific Inc.) at 0.5 µg/cm², followed by adherent culture at 37°C in a 5% CO₂ atmosphere. StemFit (registered trademark) AK02N (AJINOMOTO CO., INC.) was used as a medium, and medium exchange was carried out daily. Y-27632 (FUJIFILM Wako Pure Chemical Corporation) was added to the medium, thereby yielding a final concentration of 10 µM only during cell seeding.

Human iPS cell line 1383D6, after the adherent culture for 4 days in the manner described above, was treated with Accutase for 5 minutes to be detached from the culture surface, and the cells were dispersed into a single state by pipetting. The cells were suspended in StemFit (registered trademark) AK02N medium containing Y-27632 at a final concentration of 10µM, and some of the cells were stained with trypan blue, followed by measurement of the viable cell count. A cell suspension was prepared using StemFit (registered trademark) AK02N medium (seeding medium in suspension culture) containing Y-27632 at a final concentration of 10 µM such that the cell suspension included 2×10⁵ cells per 1 mL. Then, the cell suspension in an amount of 4 mL was seeded per well in a 6-well plate for suspension culture (Sumitomo Bakelite Co., Ltd.). The plate seeded with cells was rotated on a rotary shaker at a speed of 90 rpm along a horizontal plane in a circle with a rotation width (diameter) of 25 mm, during which the suspension culture was performed at 37°C in a 5% CO₂ environment. It was confirmed that cell aggregates were formed on day 1 of culture.

The day when the cells were seeded was defined as day 0 of culture, passage was performed on day 5 of culture, and suspension culture was performed until day 10 of culture. Medium exchange was carried out every day. As for the method of medium exchange, the entire amount of the medium containing cell masses was collected into a centrifuge tube, and the tube was allowed to stand for about 5 minutes, thereby settling the cell masses. Then, the culture supernatant was removed, and the residue was gently resuspended in StemFit (registered trademark) AK02N medium, and returned to the original well.

Upon medium exchange, Y-27632 was added to the medium at a final concentration of 5 µM on days 1 and 6 of culture, and Y-27632 was added to the medium at a final concentration of 2 µM on day 2 of culture, day 3, day 4, day 7, day 8, and day 9 of culture. Further, IWR-1-endo at a final concentration of 20 µM and LY333531 at a final concentration of 1 µM were added to the medium used for medium exchange after day 1 of culture.
On day 5 of culture, the cell masses and culture supernatant were collected from the wells into a centrifuge tube and allowed to stand for about 5 minutes, thereby settling the cell masses. Then, the culture supernatant was removed.

After adding 1 mL of Accutase to the cell masses, the cell masses were treated at 37°C for 10 minutes and then dispersed into a single-cell state by pipetting. The cells were suspended in StemFit (registered trademark) AK02N medium containing Y-27632 at a final concentration of 10µM, and some of the cells were stained with trypan blue, followed by measurement of the viable cell count. A cell suspension was prepared using StemFit (registered trademark) AK02N medium containing Y-27632 at a final concentration of 10 µM such that the cell suspension included 2×10⁵ cells per 1 mL. Then, cell seeding was performed in the same manner as in the case of day 0 to continue the suspension culture.

### <Example 2: Seeding in Suspension Culture in Presence of WNT Inhibitor>

Suspension culture was performed in the same manner as in Example 1, except that IWR-1-endo was added at a final concentration of 20 µM to the seeding medium of the suspension culture.

### <Comparative Example 1: Seeding in Suspension Culture in Presence of PKC Inhibitor>

Suspension culture was performed in the same manner as in Example 1, except that LY333531 was added at a final concentration of 1 µM to the seeding medium of the suspension culture.

### <Comparative Example 2: Seeding in Suspension Culture in Coexistence of PKCβ Inhibitor and WNT Inhibitor>

Suspension culture was performed in the same manner as in Example 1, except that IWR-1-endo was added at a final concentration of 20 µM and LY333531 was added at a final concentration of 1 µm to the seeding medium of the suspension culture.

### <Example 3: Suspension Culture with Addition of PKCβ Inhibitor after Cell Mass Formation>

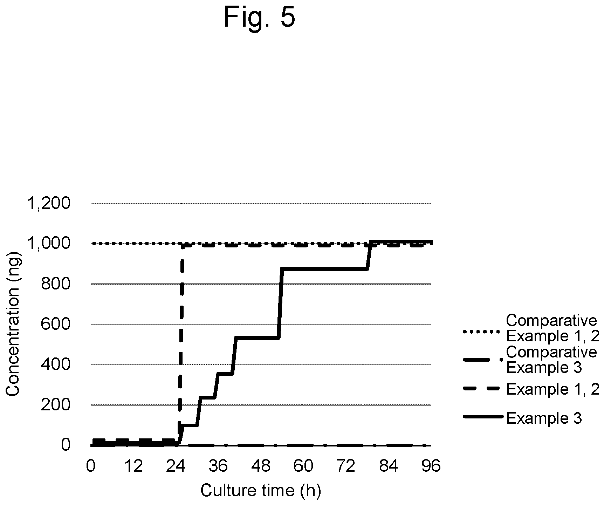

Suspension culture was performed in the same manner as in Example 2, except that the final concentration of LY333531 in the medium used for medium exchange was gradually increased, as shown in Figure 5, after day 1 of culture when cell masses were formed in the suspension culture.

### <Comparative Example 3: Suspension Culture without Addition of PKC Inhibitor>

Suspension culture was performed in the same manner as in Example 2, except that LY333531 was not added to the medium used for medium exchange after day 1 of culture.

### <Evaluation Example 3: Confirmation of Cell Mass Formation Efficiency at Start of Suspension Culture>

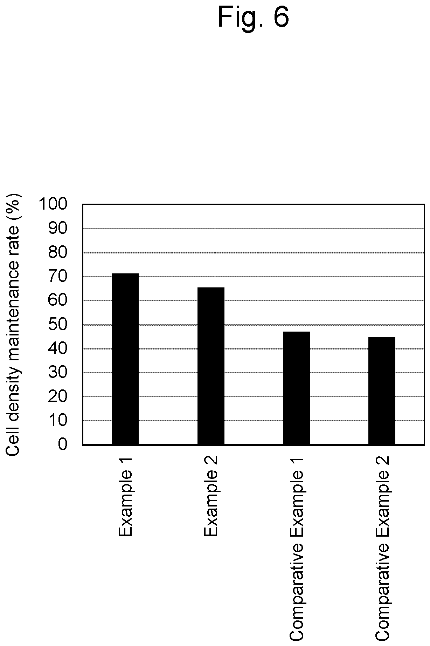

The cells cultured by the suspension culture on day 1 of culture in Examples 1 and 2 and Comparative Examples 1 and 2 were collected. The cell masses were processed into a single-cell state in the same manner described in Reference Example 2, and the number of cells was counted. Figure 6 shows the results. As shown in Figure 6, it was suggested that the inclusion of a PKCβ inhibitor during the seeding of the suspension culture reduces the cell survival rate (increases the number of dead cells) during the cell mass formation phase and/or suppress cell division immediately after seeding. In other words, it was confirmed that in order to improve productivity, it is preferable that the PKCβ inhibitor is not present at the time of seeding in the suspension culture and before cell mass formation.

### <Evaluation Example 4: Cell Growth Evaluation during Cell Growth Phase>

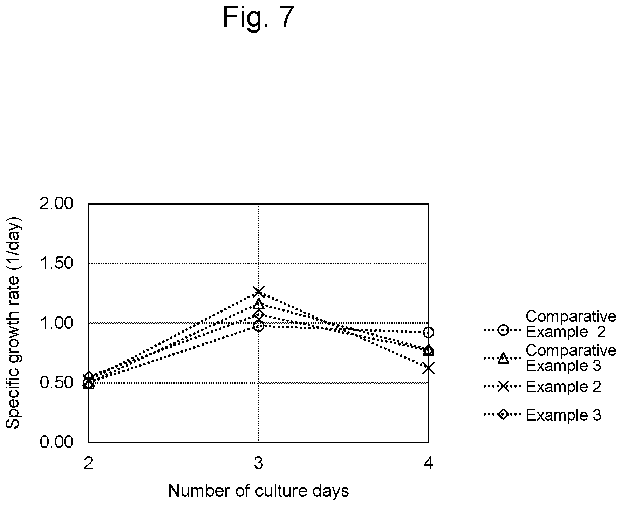

The cells cultured by the suspension culture on days 1, 2, 3, and 4 of culture in Examples 2 and 3 and Comparative Examples 2 and 3 were collected. The cell masses were processed into single-cell state in the same manner described in Reference Example 2, and the number of cells was counted. Further, based on the measured number of cells, the specific growth rate of the cells in the growth phase was calculated. Figure 7 shows the transition of the specific growth rate. The value displayed as the specific growth rate on day n of culture indicates the specific growth rate from day n-1 of culture to day n of culture. As shown in Figure 7, no difference was observed in the specific growth rate under each culture condition. Therefore, it was confirmed that even in the presence of a PKCβ inhibitor after cell mass formation, it does not induce cell death or growth inhibition and reduces productivity.

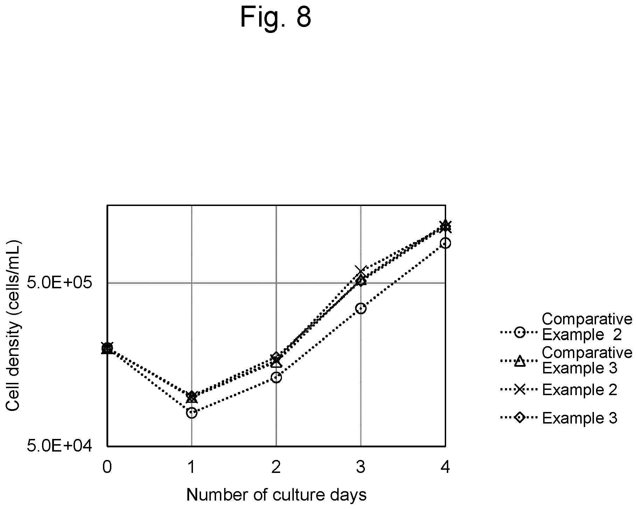

Figure 8 shows the transition of the cell density. It was suggested that the decrease in the number of cells during the cell mass formation stage, which is caused by adding a PKCβ inhibitor at the start of the suspension culture, is directly linked to a decrease in the cell yield at the end of the culture. In other words, it was assumed that it is extremely important that no PKCβ inhibitor is contained in the medium from the start of the suspension culture of the cells in a single-cell state until the formation of cell masses to avoid a decrease in the cell yield at the end of the culture.

### <Evaluation Example 5: Quantitative Real-Time PCR Analysis>

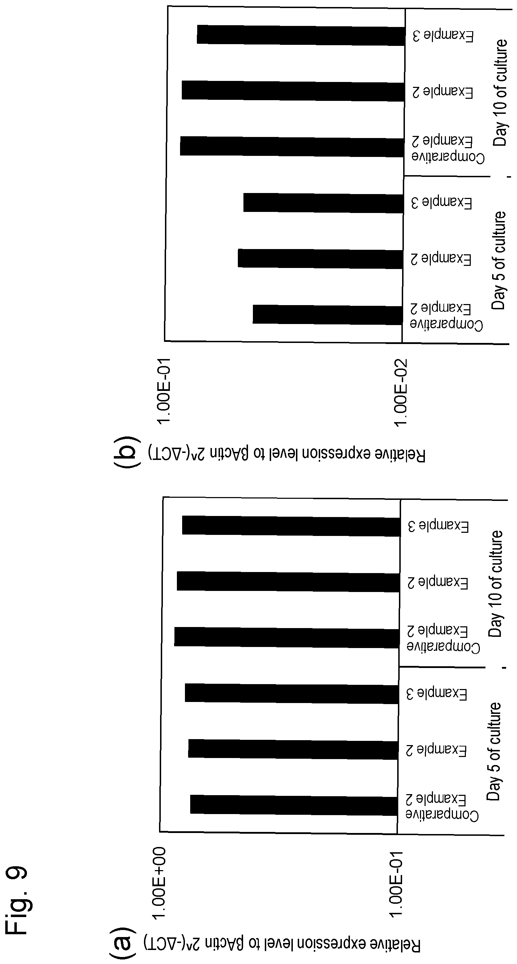

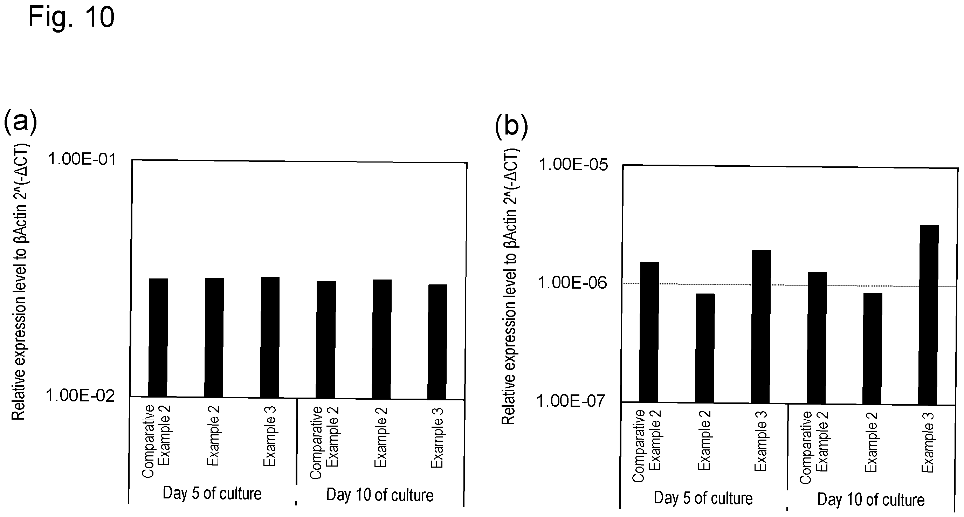

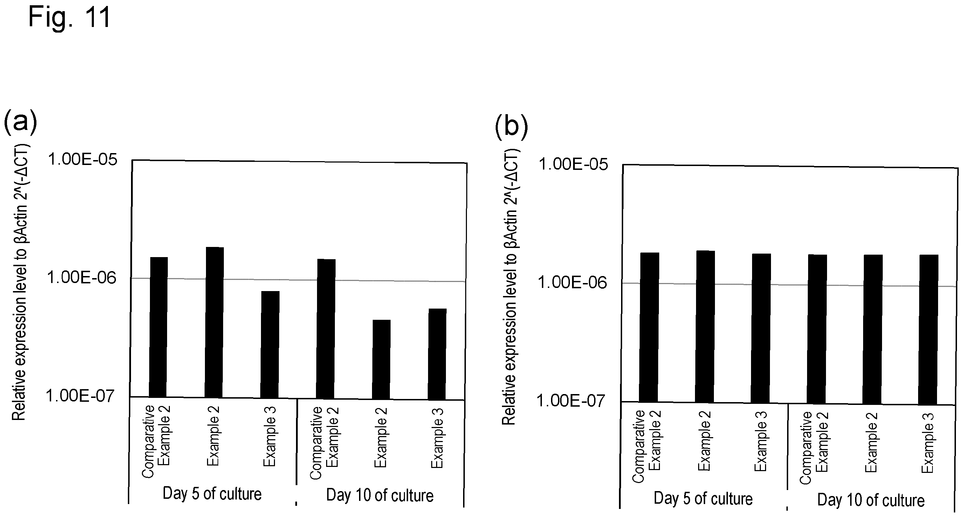

Quantitative real-time PCR analysis was performed on the cells on day 5 and day 10 of culture of Examples 2 and 3 and Comparative Example 2 in the same manner as in Evaluation Example 1. Figures 9 to 11 show the results. Figures 9(a) and 9(b) show the measurement results of the gene expressions of OCT4 and NANOG. Figures 10(a) and 10(b) show the measurement results of the gene expressions of SOX2 and TBXT. Figures 11(a) and 11(b) show the measurement results of the gene expressions of SOX17 and PAX6. It was confirmed that even the expression levels of undifferentiated markers OCT4, NANOG, and SOX2 in Examples 2 and 3 in which no PKCβ inhibitor was added at the start of the suspension culture to improve productivity, but the addition of a PKCβ inhibitor was started after cell mass formation was comparable to those in Comparative Example 2 in which a PKCβ inhibitor was added from the start of the suspension culture to suppress the differentiation. The expression levels of TBXT, a mesodermal differentiation marker, SOX17, an endodermal differentiation marker, and PAX6, an ectodermal differentiation marker, were also suppressed at comparable low levels. In other words, it was shown that by adding a PKCβ inhibitor after cell mass formation without adding a PKCβ inhibitor at the start of the culture, productivity can be improved, and the pluripotent stem cells can be cultured by the suspension culture without becoming undifferentiated.

### <Evaluation Example 6: Flow Cytometry Analysis>

Flow cytometry analysis was performed on the cells on day 5 and day 10 of culture of Examples 2 and 3 and Comparative Example 2 according to the procedure below. Cell masses were treated with Accutase and dispersed into a single-cell state by pipetting. The cells were washed with PBS (phosphate-buffered saline). Thereafter, the cells were fixed with 4% PFA (paraformaldehyde) for 20 minutes at room temperature, washed three times with PBS, and permeabilized with cold methanol at -20°C overnight. After washing three times with PBS, blocking was performed for 1 hour at room temperature with 3% FBS (fetal bovine serum)/PBS. Then, the cell sample was divided into two, and 50 µL of each was resuspended. Fluorescence-labeled anti-OCT4, anti-SOX2, and anti-NANOG antibodies were added to one of them and mixed, and a fluorescence-labeled isotype control antibody was added to the other and mixed, followed by staining at 4°C in the dark for 1 hour. Table 2 lists the antibodies used with their amounts added.

**[Table 2]**

| | Manufacturer, Model number | Amount added |
|---|---|---|
| Fluorescence-labeled anti-OCT4 | BioLegend, 653704 | 5 µL |
| Fluorescence-labeled anti-SOX2 antibody | BioLegend, 656110 | 5 µL |
| Fluorescence-labeled anti-NANOG antibody | BioLegend, 674010 | 5 µL |
| Fluorescence-labeled OCT4 isotype control antibody | BioLegend, 400314 | 0.625 µL |
| Fluorescence-labeled SOX2 isotype control antibody | BioLegend, 400129 | 0.625 µL |
| Fluorescence-labeled Nanog isotype control antibody | BioLegend, 400130 | 0.625 µL |

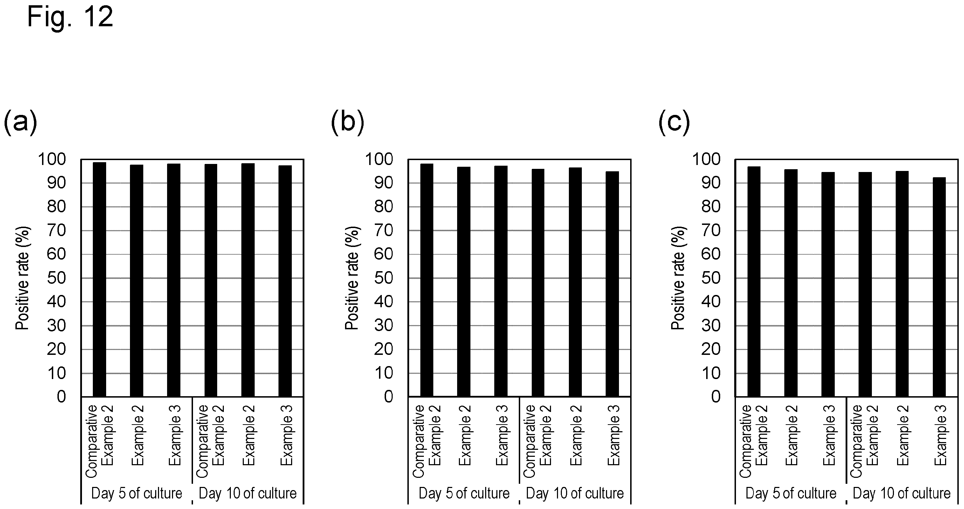

After washing once with 3% FBS (fetal bovine serum)/PBS, the cells passed through a cell strainer were analyzed with Guava easyCyte 8HT (Luminex Corporation). For the samples treated with the isotype control antibody, all fields where the cell population with the stronger fluorescence intensity was 1.0% or less in the cell populations extracted by the FSC/SSC dot plot were selected. For samples treated with the anti-OCT4, anti-SOX2, and anti-NANOG antibodies, the percentage of cells contained within the fields was calculated in the cell populations extracted by the FSC/SSC dot plot. This was defined as the proportion of cells positive for OCT4, SOX2, and NANOG. Figures 12(a), 12(b), and 12(c) show the results calculated for OCT4, SOX2, and NANOG, respectively. It was confirmed that even the positive rates for undifferentiated markers OCT4, SOX2, and NANOG in Examples 2 and 3 in which no PKCβ inhibitor was added at the start of the suspension culture, but the addition of a PKCβ inhibitor was started after cell mass formation were high at 90% or more, and they were comparable to those in Comparative Example 2 in which a PKCβ inhibitor was added from the start of the suspension culture to suppress the differentiation. It was revealed that even in a case in which pluripotent stem cells in a single-cell state are cultured by the suspension culture in a medium that does not substantially contain a PKCβ inhibitor, and after cell mass formation, they are cultured by the suspension culture in a medium containing a PKCβ inhibitor, the undifferentiated nature of the pluripotent stem cells can be maintained in the same way as in a case in which pluripotent stem cells in a single-cell state are cultured by the suspension culture in a medium containing a PKCβ inhibitor even before cell mass formation.

All publications, patents, and patent applications cited herein are incorporated herein by reference in their entirety.

## Claims

1. A method for producing pluripotent stem cells, comprising:
a step of performing suspension culture of pluripotent stem cells in a single-cell state in a first liquid medium that does not substantially contain a PKCβ inhibitor; and
a step of performing suspension culture of the pluripotent stem cells in a second liquid medium containing the PKCβ inhibitor and a WNT inhibitor after the pluripotent stem cells form a cell mass.

2. The production method according to claim 1, which is **characterized in that** the step of performing the suspension culture in the second liquid medium is carried out by adding the PKCβ inhibitor to the first liquid medium after the pluripotent stem cells form the cell mass in the step of performing the suspension culture in the first liquid medium.

3. The production method according to claim 1, which is **characterized in that** the first liquid medium is exchanged to the second liquid medium after the pluripotent stem cells form the cell mass.

4. The production method according to any one of claims 1 to 3, which is **characterized by** further comprising a step of performing adherent culture of pluripotent stem cells and a step of detaching the pluripotent stem cells after the adherent culture and processing the pluripotent stem cells into the single-cell stage, and wherein the pluripotent stem cells in the single-cell stage are cultured by the suspension culture in the first liquid medium.

5. The production method according to any one of claims 1 to 4, which is **characterized by** carrying out a step of processing a cell mass collected after the step of performing the suspension culture in the second liquid medium into cells in a single-cell state and performing suspension culture of the cells in the first liquid medium, and repeating the step of performing the suspension culture in the second liquid medium.

6. The production method according to any one of claims 1 to 5, which is **characterized in that** the first liquid medium has a concentration of the PKCβ inhibitor of less than 25 nM.

7. The production method according to any one of claims 1 to 6, which is **characterized in that** the second liquid medium has a concentration of the PKCβ inhibitor of 25 nM or more and 15 µM or less.

8. The production method according to any one of claims 1 to 7, which is **characterized in that** the first liquid medium does not substantially contain the WNT inhibitor or the first liquid medium and/or the second liquid medium has a concentration of the WNT inhibitor of 90 nM or more and 40 µM or less.

9. The production method according to any one of claims 1 to 8, which is **characterized in that** the second liquid medium has a concentration (molar concentration) ratio of the PKCβ inhibitor and the WNT inhibitor is in a range of 167:1 or more and 1:1600 or less.

10. The production method according to any one of claims 1 to 9, which is **characterized in that** the first liquid medium and the second liquid medium contain at least one selected from the group consisting of L-ascorbic acid, insulin, transferrin, selenium, and sodium hydrogen carbonate.

11. The production method according to any one of claims 1 to 10, which is **characterized in that** the first liquid medium and the second liquid medium contain FGF2 and/or TGF-β1.

12. The production method according to any one of claims 1 to 11, which is **characterized in that** the first liquid medium and the second liquid medium contain a ROCK inhibitor.

13. The production method according to claim 12, which is **characterized in that** the ROCK inhibitor is Y-27632.

14. The production method according to any one of claims 1 to 13, which is **characterized in that** the suspension culture is performed by a stirring process in the step performing the suspension culture in the first liquid medium and the step of performing the suspension culture in the second liquid medium, and a blade tip speed of a stirring blade in the step of performing the suspension culture in the second liquid medium is equal to or higher than a blade tip speed of a stirring blade in the step of performing the suspension culture in the first liquid medium.

15. The production method according to any one of claims 1 to 14, which is **characterized by** comprising a step of collecting a cell mass of the pluripotent stem cells after the step of performing the suspension culture in the second liquid medium.

16. The production method according to any one of claims 1 to 15, which is **characterized in that** the step of performing the suspension culture in the first liquid medium is carried out within 48 hours.

17. The production method according to any one of claims 1 to 16, which is **characterized in that** a proportion of cells positive for OCT4 is 90% or more, a proportion of cells positive for SOX2 is 90% or more, and a proportion of cells positive for NANOG is 90% or more among the pluripotent stem cells.

18. The production method according to any one of claims 1 to 17, which is **characterized in that** the pluripotent stem cells are ES cells and/or induced pluripotent stem cells.

19. Pluripotent stem cells or a pluripotent stem cell population produced by the production method according to any one of claims 1 to 18.
